# EUROPEAN PATENT SPECIFICATION

(11) **EP 4 025 687 B1**
(45) Date of publication and mention of the grant of the patent: **17.09.2025**
(21) Application number: 20860408.2
(22) Date of filing: 23.08.2020
(51) Int. Cl.: A61K 35/15, C12N 5/071, C12N 5/078, A61K 45/00, A61P 19/02, A61K 35/17, A61K 40/10, A61K 40/22, A61K 40/41

(54) **THERAPEUTIC APOPTOTIC CELLS FOR TREATMENT OF OSTEOARTHRITIS**
THERAPEUTISCHE APOPTOTISCHE ZELLEN ZUR BEHANDLUNG VON OSTEOARTHRITIS
CELLULES APOPTOTIQUES THÉRAPEUTIQUES POUR LE TRAITEMENT DE L'OSTÉOARTHROSE

(30) Priority: 03.09.2019 US 201962894982 P
(43) Date of publication of application: 13.07.2022
(73) Proprietor: Enlivex Therapeutics R&D Ltd, 7403618 Nes-Ziona (IL)
(72) Inventor: MEVORACH, Dror, 9574630 Jerusalem (IL); NOVIK, Shai, 4721249 Ramat Hasharon (IL)
(74) Representative: Pearl Cohen Zedek Latzer Baratz UK LLP
(86) International application number: PCT/IL2020/050919
(87) International publication number: WO 2021/044405

(56) References cited:
- WO-A1-2014/087408
- WO-A2-2006/071773
- US-A1- 2018 094 244
- US-A1- 2018 104 277
- MEVORACH DROR ET AL: "Early Apoptotic Cells (ApoCell) As Prophylaxis of Graft-Versus-Host Disease in Myeloablative HLA-Matched Allogeneic Bone Marrow Transplantation Is Safe and Effective: 1 Year Follow-up", BLOOD, AMERICAN SOCIETY OF HEMATOLOGY, US, vol. 124, no. 21, 14 November 2014 (2014-11-14), pages 5866, XP086742083, ISSN: 0006-4971, [retrieved on 20210625], DOI: 10.1182/BLOOD.V124.21.5866.5866
- BONNEFOY FRANCIS ET AL: "Apoptotic cell infusion treats ongoing collagen-induced arthritis, even in the presence of methotrexate, and is synergic with anti-TNF therapy", ARTHRITIS RESEARCH & THERAPY, vol. 18, no. 1, 11 August 2016 (2016-08-11), XP093068886, DOI: 10.1186/s13075-016-1084-0
- PATEL DIPAK V: "Gorham's Disease or Massive Osteolysis", CLINICAL MEDICINE & RESEARCH, vol. 3, no. 2, 1 May 2005 (2005-05-01), pages 65 - 74, XP055814979

## Description

### FIELD OF DISCLOSURE

Disclosed herein are compositions comprising mononuclear-enriched early apoptotic cell populations for use in methods of treating osteoarthritis in a subject. Direct administration of a mononuclear-enriched early apoptotic cell composition into a joint may reduce pain, swelling, cartilage degeneration, and bone erosion.

### BACKGROUND

The *in vitro* and *in vivo* properties of apoptotic cells suggest their potential use in a broad range of inflammatory and autoimmunity and conditions associated with cytokine storm. Multiple mechanisms are used by apoptotic cells to create an immune homeostatic anti-inflammatory state in macrophages and dendritic cells (DCs). These include direct binding to phosphatidyl serine (PtdSer) and indirect binding to Tyro3, Axl, and Mer (TAM) receptors, as well as signaling via opsonins/bridging molecules that use additional integrins and Scavenger Receptors (ScRs) to inhibit Toll-like receptors (TLRs) as well as NF-κb, STAT1, and IFN signaling, and to activate Liver X Receptors (LXR), Suppression of Cytokine Signaling (SOCS 1/3), Peroxisome Proliferator-Activated Receptors (PPAR)-δ, and hepatic growth factor (HGF). The sum of these events leads to downregulation of the inflammatory characteristics of macrophages and DCs, repair, and peripheral tolerance.

Indeed, autoimmune and autoinflammatory conditions, including type 1 diabetes in non-obese diabetic mice, experimental auto immune encephalomyelitis, arthritis, colitis, pulmonary fibro sis, fulminant hepatitis, contact hypersensitivity, acute- and chronic-graft rejection, hematopoietic cell engraftment, acute graft-versus-host disease (GvHD), and reduction of infarction size after acute myocardial infarction, were treated quite successfully by apoptotic cell infusion (Saas et al., (2016) "Concise Review: Apoptotic Cell-Based Therapies-Rationale, Preclinical Results and Future Clinical Developments." Stem Cells Volume 34(6): 1464-1473). In the majority of the studies reviewed, administration of the apoptotic cells was systemic, wherein the study specifically examining treatment of arthritis administration was by intravenous or intraperitoneal injection (*i.v*. or *i.p.,* respectively).

Among the autoimmune and autoinflammatory conditions, rheumatoid arthritis (RA) models were included in the review by Saas et al. (Saas *et al., ibid*)*.* In the collagen induced arthritis (CIA) model, Gray *et al.* (M. Gray, K. Miles, D. Salter, D. Gray, and J. Savill. (2007) "Apoptotic cells protect mice from autoimmune inflammation by the induction of regulatory B cells." Proc Natl Acad Sci U S A. Aug 28; 104(35): 14080-14085.) identified the mechanism of action in RA. The data presented in Grey *et al.,* indicated that, in two different model systems and three different strains of mice, the administration of apoptotic cells was able to affect splenic B cells such that they secreted IL-10 and enhanced secretion of the immunosuppressive cytokine IL-10 from antigen-specific effector CD4+ T cells. In addition, they inhibited the severity of inflammatory arthritis and the generation of pathogenic autoantibody despite immunization in the presence of a powerful adjuvant, CFA. Thus, administration of apoptotic cells or the passive transfer of B cells from AC-treated mice protected the mice from CIA. Apoptotic cells themselves did not secrete immunosuppressive cytokines, such as IL-10 or TGF-β, and were unable to affect the progression of a passively induced arthritis. This demonstrates that apoptotic cells are able to directly affect B cell function and cytokine production inducing regulatory B cells. Furthermore, in the CIA model they found that injecting apoptotic cells by the *i.v.* or *i.p.* route was protective because both deliver apoptotic cells to the spleen. The authors clearly suggest that if the chosen route of administration does not deliver apoptotic cells to the spleen such that the apoptotic cells can interact with B cells, protection is unlikely to be elicited.

In additional studies using experimental streptococcal cell wall (SCW)-induced arthritis Perruche *et al.* (2009) (Sylvain Perruche, Philippe Saas, and Wanjun Chen. "Apoptotic cell-mediated suppression of streptococcal cell wall-induced arthritis is associated with alteration of macrophage function and local regulatory T-cell increase: a potential cell-based therapy?" Arthritis Res Ther. 11(4): R104.), demonstrated again that the apoptotic cell effect in RA model is via lymphoid organ and via *i.p* (equivalent to I.V.) route of administration. They showed that only apoptotic cells administered by *i.p.* injection profoundly suppressed joint swelling and destruction typically observed during the acute and chronic phases of SCW-induced arthritis. This was suggested to be a result of higher Foxp3+ Tregs in the lymphoid organs, especially in the draining lymph nodes.

Last, but not least, Notley *et al.* (Notley CA1, Brown MA, Wright GP, Ehrenstein MR. (2011) "Natural IgM is required for suppression of inflammatory arthritis by apoptotic cells." J Immunol. Apr 15;186(8):4967-72. doi: 10.4049/jimmunol.1003021.) using Ag-induced model of inflammatory arthritis, showed that the enhanced production of IL-10 by T cells from draining lymph nodes and splenic marginal zone B cells, driven by the systemic infusion of apoptotic cells, was abrogated in the absence of natural IgM. Apoptotic cells were administered by i.v. injection and were present shortly after administration in the splenic marginal zone.

The clearance of dying cells is vital for re-establishing tolerance during inflammation and has potent immunoregulatory consequences. Because natural IgM plays a key role in the removal of apoptotic cells, Notely *et al.* (ibid) investigated whether the immune modulatory properties of apoptotic cells depended on its presence. Using an Ab-independent, Ag-induced model of inflammatory arthritis, they tested whether natural IgM is essential for the arthritis-suppressing properties of apoptotic cells. Whereas administration of apoptotic cells reduced joint inflammation and damage in normal mice accompanied by suppression of the Th17 response, no protection was afforded in secreted IgM-deficient (Sµ(-)) mice. The enhanced production of IL-10 by T cells from draining lymph nodes and splenic marginal zone B cells, driven by the infusion of apoptotic cells, was abrogated in the absence of natural IgM. Apoptotic cells were present shortly after administration in the splenic marginal zone, concluding that natural IgM is a critical factor in a chain of events triggered by the administration of apoptotic cells that promote splenic IL-10-secreting B and T cells and restrain the development of inflammation.

Taken together these results suggest that apoptotic cells modify RA via splenic effect on B and T cells and thus modify autoimmunity. A direct intra-joint effect on a non-autoimmune condition is not expected.

Osteoarthritis is the most prevalent musculoskeletal disorder and one for which there is no disease modifying therapy available at present. The current understanding of the disease mechanism of osteoarthritis is limited owing to a lacuna of knowledge about the development and maintenance of articular cartilage that is affected during osteoarthritis. During osteoarthritis, articular cartilage expresses markers for transient cartilage differentiation. Moreover, blocking transient cartilage differentiation is sufficient for halting the progression of experimental osteoarthritis. A developmental biology inspired approach that combines restoration of tissue microenvironment, supplementation with engineered cartilage and built in mechanism to prevent transient cartilage differentiation could be an avenue for developing a disease modifying therapy for osteoarthritis. Non-steroidal anti-inflammatory drugs which might mitigates pain, do not arrest the progressive degeneration of articular cartilage. Therefore, non-steroidal anti-inflammatory drugs or corticosteroids are not considered of a therapeutic benefit in osteoarthritis, and in that regard apoptotic cells as an immune modulator were not really expected to modify osteoarthritis.

"Vanishing bone disease" is a clinical presentation mainly involving the hips and shoulders, but other joints as well, that could evolve mainly from rapidly destructive arthritis due to erosive osteoarthritis (Mavrogenis AF, Flevas DA, Panagopoulos GN, et al. (2015) "Rapid destructive arthritis of the hip revisited." Eur J Orthop Surg Traumatol. 25:1115-20) or spontaneous bone osteolysis due to proliferation of lymphangiomatous tissue, i.e, the Gorham-stout variant (Dellinger MT, Garg N, Olsen BR. (2014) "Viewpoints on vessels and vanishing bones in Gorham-Stout disease." Bone 63:47-52). No effective treatment has been identified for these conditions.

Thus, there remains an unmet need for compositions useful in the treatment of non-autoimmune conditions such as osteoarthritis and vanishing bone disease, including for the treatment for pain reduction, reduction of inflammation, reduction of swelling, inhibition or slowing the progressive degeneration of articular cartilage, inhibition or slowing of erosion of bone tissue, and treatment for increased movement including increased range of movements. Apoptotic cell infusion directly into a joint may present a novel and safe treatment for rebalancing the immune response in the bone and joint.

US2018/094244 discloses a combination immune therapy and cytokine control therapy for cancer treatment, wherein the subjects are administered compositions including apoptotic cells or apoptotic cell supernatants.

WO2006/071773 discloses cells derived from postpartum tissue and methods for their isolation and induction to differentiate to cells of a chondrogenic or osteogenic phenotype. The postpartum-derived cells of the invention and products related thereto have therapeutic applications in the treatment of bone and cartilage conditions such as osteoarthritis.

US2018/104277 discloses therapeutic pooled blood apoptotic cell preparations and uses thereof in treating an immune disease, an inflammatory disease, an autoimmune disease, or infertility in a subject.

V. Patel Dipak et al. disclose the treatment of Gorham's disease with chylothorax that was treated with radiation therapy (Clinical Medicine & Research, vol 3, no. 2, 1 May 2005, pages 65-74).

### SUMMARY

The present invention provides a composition comprising a mononuclear-enriched early apoptotic cell population comprising more than 40% Annexin V and less than 15% PI positive cells, wherein the cells are irradiated following the induction of apoptosis, for use in a method of treating osteoarthritis in a human subject by administering the composition directly into a joint of the subject.

In an embodiment, treating osteoarthritis comprises pain reduction, reduction of inflammation, reduction of swelling, inhibition of progressive degeneration of articular cartilage, reduction of progressive degeneration of articular cartilage, improving a quality of life, or any combination thereof.

In an embodiment, the use increases movement in the joint, wherein increased movement comprises increased range of movement or increased movement with reduced pain, or a combination thereof.

In an embodiment, the joint comprises a synovial joint.

In an embodiment, the synovial joint comprises a knee joint, a hip joint, a shoulder joint, a joint between neck vertebrae, an elbow joint, an ankle joint, a wrist joint, a finger joint, a toe joint, or a thumb joint, a hand joint, a foot joint, or a combination thereof.

In an embodiment, the composition is formulated for direct administration into the joint as an infusion or injection.

In an embodiment, the early apoptotic cell population comprises a pooled population of early apoptotic cells.

In an embodiment, the pooled early apoptotic cell population comprises apoptotic cells prepared from a single donor or from multiple donor mononuclear cells.

In an embodiment, the administering comprises a single administration or multiple administrations of the early apoptotic cell population.

In an embodiment, the multiple administration comprises daily or weekly administrations.

In an embodiment, the composition is formulation to comprise a dose of between about 10 x 10⁶ ±20% to 1 x 10⁹ ±20% early apoptotic cells/kg subject.

### BRIEF DESCRIPTION OF THE DRAWINGS

The subject matter regarded as the invention is particularly pointed out and distinctly claimed in the appended claims. The invention, however, both as to organization and method of operation, together with objects, features, and advantages thereof, may best be understood by reference to the following detailed description when read with the accompanying drawings in which:

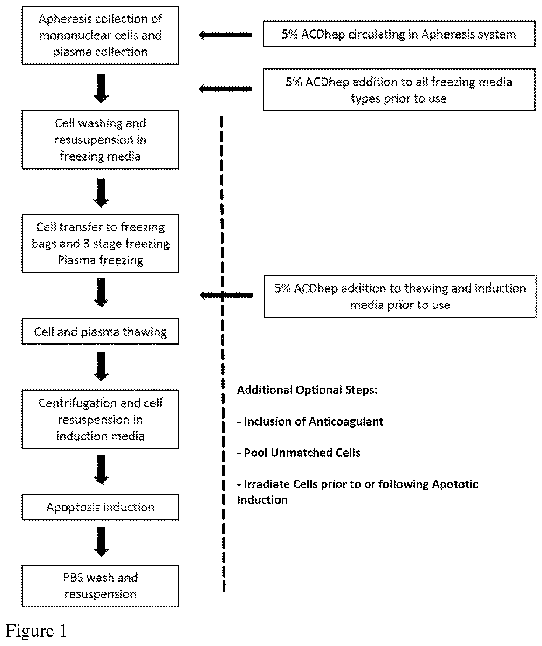

**Figure 1** presents a flow chart of the steps during a manufacturing process of early apoptotic cell populations, wherein anti-coagulants were included in the process (See Examples 1 and 2 for details of different embodiments). The mononuclear cells collected could be autologous or allogeneic, wherein non-matched mononuclear cells are used in some instances. An additional step includes irradiating the cells and pooling unmatched cells if multiple sources of cells used.

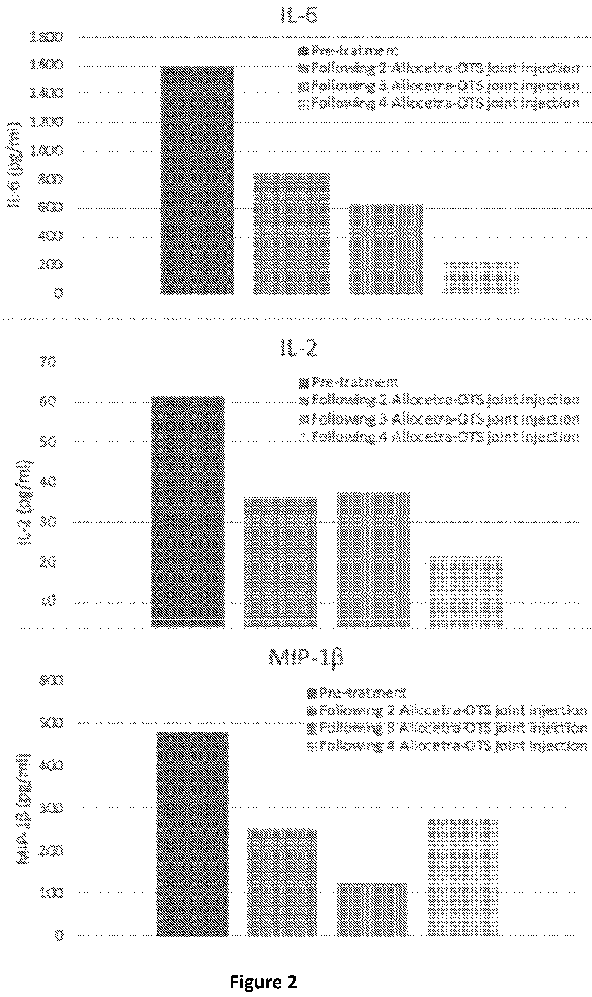

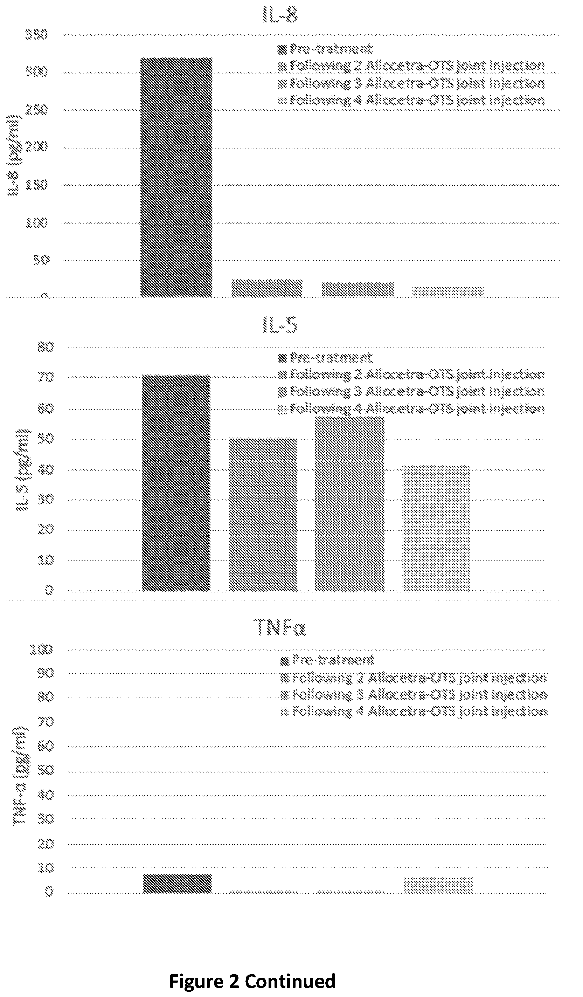

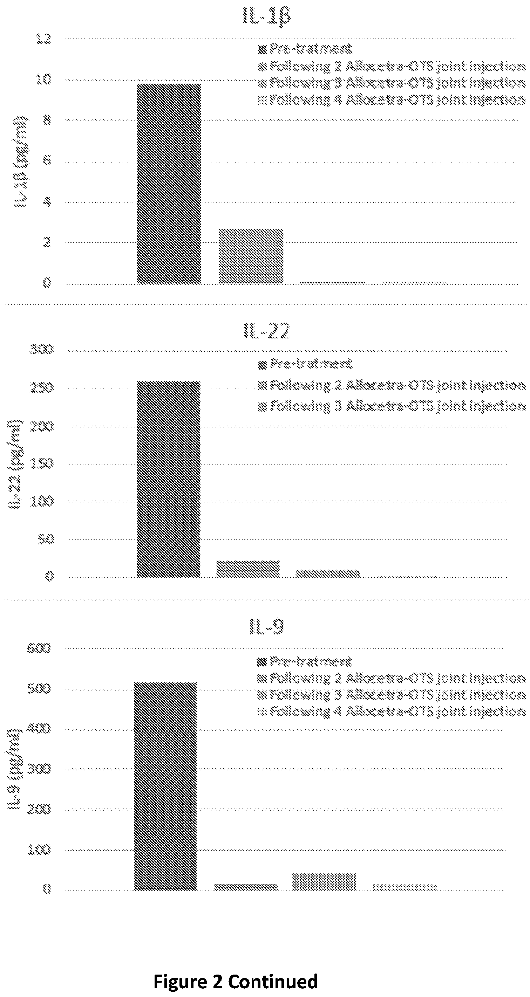

**Figure 2** presents a series of bar graphs showing cytokine/chemokine levels in the right shoulder joint before and after treatment with early apoptotic cells. Cytokines/chemokines measurement was performed via Luminex MAGPIX system and analysis performed using Milliplex software (See Example 4 below).

### DETAILED DESCRIPTION

The present invention provides a composition comprising a mononuclear-enriched early apoptotic cell population comprising more than 40% Annexin V and less than 15% PI positive cells, wherein the cells are irradiated following the induction of apoptosis, for use in a method of treating osteoarthritis in a human subject by administering the composition directly into a joint of the subject.

Unlike rheumatoid arthritis, osteoarthritis and vanishing bone disease are not considered inflammatory autoimmune diseases or disorders. The compositions described herein may be useful in addressing multiple types of treatment, not restricted to treating autoimmunity. In some embodiments, compositions for use according to the present invention by-pass the systemic immune system and the necessity of any response thereof as a required element of the effective therapy.

### Apoptotic Cells

The production of early apoptotic cells ("ApoCells") for use in compositions according to the present invention has been described in WO 2014/087408 and is described in brief in Example 1 below. Early apoptotic cells for use in compositions as disclosed herein can be produced in any way that is known in the art. In another embodiment, early apoptotic cells for use in compositions disclosed herein are autologous with a subject undergoing therapy. In another embodiment, early apoptotic cells for use in compositions disclosed herein are allogeneic with a subject undergoing therapy. In another embodiment, early apoptotic cells for use in compositions disclosed herein are not matched to a subject undergoing therapy, in other words they are "Off the shelf" (OTS). In another embodiment, a composition comprising early apoptotic cells comprises early apoptotic cells as disclosed herein or as is known in art.

A skilled artisan would appreciate that the term "autologous" may encompass a tissue, cell, nucleic acid molecule or polypeptide in which the donor and recipient is the same person.

A skilled artisan would appreciate that the term "allogeneic" may encompass a tissue, cell, nucleic acid molecule or polypeptide that is derived from separate individuals of the same species. In some embodiments, allogeneic donor cells are genetically distinct from the recipient.

A source of cells for the early apoptotic population comprises mononuclear-enriched cells. Obtaining a mononuclear-enriched cell composition may be achieved by leukapheresis. A skilled artisan would appreciate that the term "leukapheresis" may encompass an apheresis procedure in which leukocytes are separated from the blood of a donor. The blood of a donor undergoes leukapheresis and thus a mononuclear-enriched cell composition is obtained. It is to be noted, that the use of at least one anticoagulant during leukapheresis is required, as is known in the art, in order to prevent clotting of the collected cells.

The leukapheresis procedure is configured to allow collection of mononuclear-enriched cell composition. Cell collections obtained by leukapheresis may comprise at least 65% mononuclear cells, at least 70%, or at least 80% mononuclear cells. Blood plasma from the cell-donor may be collected in parallel to obtaining of the mononuclear-enriched cell composition. About 300-600ml of blood plasma from the cell-donor may be collected in parallel to obtain the mononuclear-enriched cell composition. Blood plasma may be collected in parallel to obtain the mononuclear-enriched cell composition and may be used as part of the freezing and/or incubation medium. Additional detailed methods of obtaining an enriched mononuclear population of apoptotic cells for use according to the invention may be found in WO 2014/087408.

The early apoptotic cells for use disclosed herein may comprise at least 85% mononuclear cells. The early apoptotic cells for use disclosed herein may contain at least 85% mononuclear cells, 90% mononuclear cells or alternatively over 90% mononuclear cells. The early apoptotic cells for use disclosed herein may comprise at least 90% mononuclear cells. The early apoptotic cells for use disclosed herein may comprise at least 95% mononuclear cells.

It is to be noted that while the mononuclear-enriched cell preparation at cell collection may comprise at least 65%, preferably at least 70%, most preferably at least 80% mononuclear cells, the final pharmaceutical population following the production method of the early apoptotic cells for use according to the present invention, comprises at least 85%, preferably at least 90%, most preferably at least 95% mononuclear cells.

The mononuclear-enriched cell preparation used for production of the composition of the early apoptotic cells comprises at least 50% mononuclear cells at cell collection. Also disclosed herein is a method for producing the pharmaceutical population for use according to the present invention, wherein the method comprises obtaining a mononuclear-enriched cell preparation from the peripheral blood of a donor, the mononuclear-enriched cell preparation comprising at least 50% mononuclear cells. Further disclosed herein is a method for producing the pharmaceutical population wherein the method comprises freezing a mononuclear-enriched cell preparation comprising at least 50% mononuclear cells.

The cell preparation may comprise at least 85% mononuclear cells, wherein at least 40% of the cells in the preparation are in an early-apoptotic state, and wherein at least 85% of the cells in the preparation are viable cells. The apoptotic cell preparation may comprise no more than 15% CD15^{high} expressing cells.

A skilled artisan would appreciate that the term "early-apoptotic state" may encompass cells that show early signs of apoptosis without late signs of apoptosis. Examples of early signs of apoptosis in cells include exposure of phosphatidylserine (PS) and the loss of mitochondrial membrane potential. Examples of late events include propidium iodide (PI) admission into the cell and the final DNA cutting. In order to document that cells are in an "early apoptotic" state, PS exposure detection by Annexin-V and PI staining are used, and cells that are stained with Annexin V but not with PI or with only minimal PI staining are considered to be "early apoptotic cells" (An⁺PI⁻). Minimal IP staining comprising less than or equal to (≤)15% PI+ cells within the population of cells. Minimal IP staining may comprise ≤ 10% PI+ cells within the population of cells. In some embodiments, minimal IP staining may comprise ≤ 5% PI+ cells within the population of cells. Cells that are stained by both Annexin-V FITC and high PI may be considered to be "late apoptotic cells". High IP staining may comprise greater than (>) 15% PI+ cells within the population of cells. High IP staining may comprise greater than or equal to (≥) 16% PI+ cells within the population of cells. Cells that do not stain for either Annexin-V or PI may be considered non-apoptotic viable cells.

At least 40% of the cells in a preparation may be in an early apoptotic state. At least 45% of the cells in a preparation may be in an early apoptotic state. At least 50% of the cells in a preparation may be in an early apoptotic state. At least 55% of the cells in a preparation may be in an early apoptotic state. At least 60% of the cells in a preparation may be in an early apoptotic state. At least 65% of the cells in a preparation may be in an early apoptotic state. At least 70% of the cells in a preparation may be in an early apoptotic state. At least 75% of the cells in a preparation may be in an early apoptotic state. At least 80% of the cells in a preparation may be in an early apoptotic state. At least 85% of the cells in a preparation may be in an early apoptotic state. At least 90% of the cells in a preparation may be in an early apoptotic state. At least 95% of the cells in a preparation may be in an early apoptotic state.

An early apoptotic cell preparation may comprise less than or equal to (≤) 15% PI⁺ cells. An early apoptotic cell preparation may comprise ≤ 10% PI⁺ cells. An early apoptotic cell preparation may comprise ≤ 9% PI⁺ cells. An early apoptotic cell preparation may comprise ≤ 8% PI⁺ cells. An early apoptotic cell preparation may comprise ≤ 7% PI⁺ cells. An early apoptotic cell preparation may comprise ≤ 6% PI⁺ cells. An early apoptotic cell preparation may comprise ≤ 5% PI⁺ cells. An early apoptotic cell preparation may comprise ≤ 4% PI⁺ cells. An early apoptotic cell preparation may comprise ≤ 3% PI⁺ cells. An early apoptotic cell preparation may comprise ≤ 2% PI⁺ cells. An early apoptotic cell preparation may comprise ≤ 1% PI⁺ cells.

At least 40% of the cells in a preparation may be in an early apoptotic state (An⁺), wherein ≤ 15%, 10%, 9%, 8%, 7%, 6%, 5%, 4%, 3%, 2%, 1%, or 0% of the cells are PI⁺. At least 45% of the cells in a preparation may be in an early apoptotic state (An⁺), wherein ≤ 15%, 10%, 9%, 8%, 7%, 6%, 5%, 4%, 3%, 2%, 1%, or 0% of the cells are PI⁺. At least 50% of the cells in a preparation may be in an early apoptotic state (An⁺), wherein ≤ 15%, 10%, 9%, 8%, 7%, 6%, 5%, 4%, 3%, 2%, 1%, or 0% of the cells are PI⁺. At least 55% of the cells in a preparation may be in an early apoptotic state (An⁺), wherein ≤ 15%, 10%, 9%, 8%, 7%, 6%, 5%, 4%, 3%, 2%, 1%, or 0% of the cells are PI⁺. At least 60% of the cells in a preparation may be in an early apoptotic state (An⁺), wherein ≤15%, 10%, 9%, 8%, 7%, 6%, 5%, 4%, 3%, 2%, 1%, or 0% of the cells are PI⁺. At least 65% of the cells in a preparation may be in an early apoptotic state (An⁺), wherein ≤ 15%, 10%, 9%, 8%, 7%, 6%, 5%, 4%, 3%, 2%, 1%, or 0% of the cells are PI⁺. At least 70% of the cells in a preparation may be in an early apoptotic state (An⁺), wherein ≤ 15%, 10%, 9%, 8%, 7%, 6%, 5%, 4%, 3%, 2%, 1%, or 0% of the cells are PI⁺. At least 75% of the cells in a preparation may be in an early apoptotic state (An⁺), wherein ≤ 15%, 10%, 9%, 8%, 7%, 6%, 5%, 4%, 3%, 2%, 1%, or 0% of the cells are PI⁺. At least 80% of the cells in a preparation may be in an early apoptotic state (An⁺), wherein ≤ 15%, 10%, 9%, 8%, 7%, 6%, 5%, 4%, 3%, 2%, 1%, or 0% of the cells are PI⁺. At least 85% of the cells in a preparation may be in an early apoptotic state (An⁺), wherein < 15% or ≤14%, 10%, 9%, 8%, 7%, 6%, 5%, 4%, 3%, 2%, 1%, or 0% of the cells are PI⁺. At least 90% of the cells in a preparation may be in an early apoptotic state (An⁺), wherein < 10%, or ≤ 9%, 8%, 7%, 6%, 5%, 4%, 3%, 2%, 1%, or 0% of the cells may be PI⁺. At least 95% of the cells in a preparation may be in an early apoptotic state (An⁺), wherein < 5%, ≤4%, 3%, 2%, 1%, or 0% of the cells are PI⁺.

A skilled artisan would appreciate that in some embodiments the terms "apoptotic cell", "early apoptotic cell", "Allocetra", Autocetra", "ALC", and "ApoCell", and grammatical variants thereof, may be used interchangeably to represent a population of "early apoptotic cells", wherein said cell population is enriched for mononuclear cells and has unique characteristics (See for example, Example 1).

Allocetra may comprise a population of early apoptotic cells obtained from a single allogeneic donor. Allocetra may comprise a population of early apoptotic cells obtained from multiple allogeneic donors. Allocetra may comprise pooled populations of early apoptotic cells obtained from multiple allogeneic donors or from cells obtained from a blood bank. Allocetra may comprise pooled population of early apoptotic cells obtained from the same allogeneic donor. The term "Allocetra" may be used interchangeably with the term "Allocetra-OTS". The terms "Allocetra" and "Allocetra-OTS" may encompass mononuclear early apoptotic cells, prepared as described in Example 1, independent of the source of said cells.

Autocetra may comprise a population of early apoptotic cells obtained as a single donation from an autologous donor. Autocetra may comprise a population of early apoptotic cells obtained as multiple donations from an autologous donor. Autocetra may comprise an irradiated population of early apoptotic cells. The term "Autocetra" may be used interchangeably with the term "Autocetra-OTS".

Early apoptotic cells may be HLA matched to a recipient of a composition comprising the early apoptotic cells (a subject in need). Early apoptotic cells are not matched to a recipient of a composition comprising the early apoptotic cells (a subject in need). Irradiated not matched cells may be termed "Allocetra-OTS" or "ALC-OTS".

In this invention, the composition comprises cells in an early apoptotic state. The apoptotic cells may comprise cells wherein at least 90% of said cells are in an early apoptotic state, wherein at least 80% of said cells are in an early apoptotic state, wherein at least 70% of said cells are in an early apoptotic state, wherein at least 60% of said cells are in an early apoptotic state, or wherein at least 50% of said cells are in an early apoptotic state.

The composition comprising apoptotic cells may further comprise an anti-coagulant.

Early apoptotic cells may be stable. A skilled artisan would appreciate that stability encompasses maintaining early apoptotic cell characteristics over time, for example, maintaining early apoptotic cell characteristics upon storage at about 2-8°C. Stability may comprise maintaining early apoptotic cell characteristic upon storage at freezing temperatures, for example temperatures at or below 0°C.

The mononuclear-enriched cell population obtained according to the production method of the early apoptotic cells may undergoes freezing in a freezing medium. The freezing may be gradual. Following collection, the cells may be maintained at room temperature until frozen. The cell-preparation may undergo at least one washing step in washing medium following cell-collection and prior to freezing.

As used herein, the terms "obtaining cells" and "cell collection" may be used interchangeably. The cells of the cell preparation may be frozen within 3-6 hours of collection, or frozen within up to 6 hours of cell collection, or frozen within 1, 2, 3, 4, 5, 6, 7, 8 hours of collection. Alternatively, the cells of the cell preparations may be frozen up to 8, 12, 24, 48, 72 hours of collection. Following collection the cells may be maintained at 2-8°C until frozen.

Freezing for the production of an early apoptotic cell population may comprise freezing the cell preparation at about -18°C to -25°C followed by freezing the cell preparation at about - 80°C and finally freezing the cell preparation in liquid nitrogen until thawing. Freezing for the production of an early apoptotic cell population may comprise freezing the cell preparation at about -18°C to -25°C for at least 2 hours, freezing the cell preparation at about -80°C for at least 2 hours and finally freezing the cell preparation in liquid nitrogen until thawing. The cells may be kept in liquid nitrogen for at least 8, 10 or 12 hours prior to thawing. The cells of the cell preparation may be kept in liquid nitrogen until thawing and incubation with apoptosis-inducing incubation medium. The cells of the cell preparation may be kept in liquid nitrogen until the day of hematopoietic stem cell transplantation. In some examples, the time from cell collection and freezing to preparation of the final population may be between 1-50 days, alternatively between 6-30 days. The cell preparation may be kept in liquid nitrogen for longer time periods, such as at least several months.

Freezing for the production of an early apoptotic cell population may comprise freezing the cell preparation at about -18°C to -25°C for at least 0.5, 1, 2, 4 hours, or freezing the cell preparation at about -18°C to -25°C for about 2 hours, or freezing the cell preparation at about - 80°C for at least 0.5, 1, 2, 4, 12 hours.

The mononuclear-enriched cell composition may remain frozen at least 1, 2, 3, 4, 5, 6, 7, 8, 9, 10, 11, 12, 20 months, or may remain frozen at least 0.5, 1, 2, 3, 4, 5 years. The mononuclear-enriched cell composition may remain frozen for at least 20 months.

The mononuclear-enriched cell composition may be frozen for at least 8, 10, 12, 18, 24 hours. Freezing the mononuclear-enriched cell composition may be for a period of at least 8 hours, or may be frozen for at least about 10 hours, or for at least about 12 hours, or for about 12 hours. The total freezing time of the mononuclear-enriched cell composition (at about -18°C to -25°C, at about -80°C and in liquid nitrogen) may be at least 8, 10, 12, 18, 24 hours.

Freezing may at least partly induce the early-apoptotic state in the cells of the mononuclear-enriched cell composition. The freezing medium may comprise RPMI 1640 medium comprising L-glutamine, Hepes, Hes, dimethyl sulfoxide (DMSO) and plasma. The plasma in the freezing medium may be an autologous plasma of the donor which donated the mononuclear-enriched cells of the population. The freezing medium may comprise RPMI 1640 medium comprising 2 mM L-glutamine, 10 mM Hepes, 5% Hes, 10% dimethyl sulfoxide and 20% v/v plasma.

The freezing medium may comprise an anti-coagulant. In certain possibilities, at least some of the media used during the production of an early apoptotic cell population, including the freezing medium, the incubation medium and the washing media may comprise an anti-coagulant. In certain instances, all media used during the production of an early apoptotic cell population which comprise an anti-coagulant may comprise the same concentration of anti-coagulant. In some instances, anti-coagulant is not added to the final suspension medium of the cell population.

Addition of an anti-coagulant at least to the freezing medium may improve the yield of the cell-preparation. Addition of an anti-coagulant to the freezing medium may improve the yield of the cell-preparation in the presence of a high triglyceride level. Improvement in the yield of the cell-preparation may relate to improvement in at least one of: the percentage of viable cells out of cells frozen, the percentage of early-state apoptotic cells out of viable cells and a combination thereof.

Early apoptotic cells may be stable for at least 24 hours. Early apoptotic cells may be stable for 24 hours. Early apoptotic cells are stable for more than 24 hours, for at least 36 hours, for more than 36 hours, for at least 48 hours, for 48 hours, for more than 48 hours, for at least 72 hours, for 72 hours, or may be stable for more than 72 hours.

A skilled artisan would appreciate that the term "stable" encompasses apoptotic cells that remain PS-positive (Phosphatidylserine-positive) with only a very small percent of PI-positive (Propidium iodide-positive). PI-positive cells provide an indication of membrane stability wherein a PI-positive cells permits admission into the cells, showing that the membrane is less stable. Stable early apoptotic cells may remain in early apoptosis for at least 24 hours, for at least 36 hours, for at least 48 hours, or for at least 72 hours. Stable early apoptotic cells may remain in early apoptosis for 24 hours, for 36 hours, for 48 hours, or for 72 hours. Stable early apoptotic cells may remain in early apoptosis for more than 24 hours, for more than 36 hours, for more than 48 hours, or for more than 72 hours. Stable early apoptotic cells may maintain their state for an extended time period.

An apoptotic cell population may be devoid of cell aggregates. An apoptotic cell population may be devoid of large cell aggregates. An apoptotic cell population may have a reduced number of cell aggregates compared to an apoptotic cell population prepared without adding an anticoagulant in a step other than cell collection (leukapheresis) from the donor. An apoptotic cell population or a composition thereof, may comprise an anticoagulant.

Apoptotic cells may be devoid of cell aggregates, wherein said apoptotic cells were obtained from a subject with high blood triglycerides. Blood triglycerides levels of the subject may be above 150 mg/dL. An apoptotic cell population may be devoid of cell aggregates, wherein said apoptotic cell population is prepared from cells obtained from a subject with normal blood triglycerides. Blood triglycerides levels of the subject may be equal to or below 150 mg/dL. Cell aggregates may produce cell loss during apoptotic cell production methods.

A skilled artisan would appreciate that the terms "aggregates" or "cell aggregates" may encompass the reversible clumping of blood cells under low shear forces or at stasis. Cell aggregates can be visually observed during the incubation steps of the production of the apoptotic cells. Cell aggregation can be measured by any method known in the art, for example by visually imaging samples under a light microscope or using flow cytometry.

The anti-coagulant may be selected from heparin, acid citrate dextrose (ACD) Formula A or a combination thereof.

In some methods of preparing an early apoptotic cell population and compositions thereof, an anticoagulant may be added to at least one medium used during preparation of the population. The at least one medium used during preparation of the population may be the freezing medium, the washing medium, the apoptosis inducing incubation medium, or any combinations thereof.

The anti-coagulant may be Heparin, ACD Formula A or a combination thereof. It is to be noted that other anti-coagulants known in the art may be used, such as Fondaparinaux, Bivalirudin and Argatroban.

At least one medium used during preparation of the population may contain 5% of ACD formula A solution comprising 10 U/ml heparin. In some instances, anti-coagulant is not added to the final suspension medium of the cell population. As used herein, the terms "final suspension medium" and "administration medium" are used interchangeably having all the same qualities and meanings.

At least one medium used during preparation of the population may comprise heparin at a concentration of between 0.1-2.5 U/ml. At least one medium used during preparation of the population may comprise ACD Formula A at a concentration of between 1%-15% v/v. The freezing medium may comprise an anti-coagulant. The incubation medium may comprise an anti-coagulant. In some cases, both the freezing medium and incubation medium may comprise an anti-coagulant. The anti-coagulant may be heparin, ACD Formula A or a combination thereof.

The heparin in the freezing medium may be at a concentration of between 0.1-2.5 U/ml. The ACD Formula A in the freezing medium may be at a concentration of between 1%-15% v/v. The heparin in the incubation medium may be at a concentration of between 0.1-2.5 U/ml. The ACD Formula A in the incubation medium may be at a concentration of between 1%-15% v/v. The anticoagulant may be a solution of acid-citrate-dextrose (ACD) formula A. The anticoagulant added to at least one medium used during preparation of the population may be ACD Formula A containing heparin at a concentration of 10 U/ml.

The apoptosis inducing incubation medium used in the production of an early apoptotic cell population may comprise an anti-coagulant. Both the freezing medium and apoptosis inducing incubation medium used in the production of an early apoptotic cell population may comprise an anti-coagulant. Without wishing to be bound by any theory or mechanism, in order to maintain a high and stable cell yield in different cell compositions, regardless of the cell collection protocol, addition of anti-coagulants may comprise adding the anticoagulant to both the freezing medium and the apoptosis inducing incubation medium during production of the apoptotic cell population. A high and stable cell yield within the composition may comprise a cell yield of at least 30%, preferably at least 40%, typically at least 50% cells of the initial population of cells used for induction of apoptosis.

Both the freezing medium and the incubation medium may comprise an anti-coagulant. Addition of an anti-coagulant both to the incubation medium and freezing medium may result in a high and stable cell-yield between different preparations of the population regardless of cell-collection conditions, such as the timing and/or type of anti-coagulant added during cell collection. Addition of an anti-coagulant both to the incubation medium and freezing medium may result in a high and stable yield of the cell-preparation regardless of the timing and/or type of anti-coagulant added during leukapheresis. Production of the cell-preparation in the presence of a high triglyceride level may result in a low and/or unstable cell-yield between different preparations. Producing the cell-preparation from the blood of a donor having high triglyceride level may result in a low and/or unstable cell-yield of the cell preparation. The term "high triglyceride level" may refer to a triglyceride level which is above the normal level of a healthy subject of the same sex and age. The term "high triglyceride level" may refer to a triglyceride level above about 1.7 mM/liter. As used herein, a high and stable yield may refer to a cell yield in the population which is high enough to enable preparation of a dose which will demonstrate therapeutic efficiency when administered to a subject. Therapeutic efficiency may refer to the ability to treat, prevent or ameliorate an immune disease, an autoimmune disease or an inflammatory disease in a subject. A high and stable cell yield may be a cell yield of at least 30%, possibly at least 40%, typically at least 50% of cells in the population out of cells initially frozen.

In case the cell-preparation is obtained from a donor having a high triglyceride level, the donor may take at least one measure selected from: taking triglyceride-lowering medication prior to donation, such as: statins and/or bezafibrate, fasting for a period of at least 8, 10, 12 hours prior to donation, eating an appropriate diet to reduce blood triglyceride level at least 24, 48, 72 hours prior to donating or any combination thereof.

Cell yield in the population may relate to cell number in the composition out of the initial number of cells subjected to apoptosis induction. As used herein, the terms "induction of early apoptotic state" and "induction of apoptosis" may be used interchangeably.

The mononuclear-enriched cell composition may be incubated in incubation medium following freezing and thawing. There may be at least one washing step between thawing and incubation. As used herein, the terms "incubation medium" and "apoptosis inducing incubation medium" may be used interchangeably. The incubation medium may comprise RPMI 1640 medium supplemented with L-glutamine, Hepes methylprednisolone and plasma. The washing medium may comprise 2 mM L-glutamine, 10 mM Hepes and 10% v/v blood plasma. The blood plasma in in the incubation medium may be derived from the same donor from whom the cells of the cell preparations are derived. The blood plasma may be added to the incubation medium on the day of incubation. The incubation is performed at 37°C and 5% CO₂.

The incubation medium may comprise methylprednisolone. The methylprednisolone within the incubation medium may further induce the cells in the mononuclear-enriched cell composition to enter an early-apoptotic state. The cells in the mononuclear-enriched cell composition may be induced to enter an early-apoptotic state both by freezing and incubating in the presence of methylprednisolone. The production of an early apoptotic cell population advantageously may allow induction of an early-apoptosis state substantially without induction of necrosis, wherein the cells remain stable at said early-apoptotic state for about 24 hours following preparation.

The incubation medium may comprise methylprednisolone at a concentration of about 10-100 µg/ml. The incubation medium may comprise methylprednisolone at a concentration of about 40-60 µg/ml, alternatively about 45-55 µg/ml. The incubation medium may comprise methylprednisolone at a concentration of 50 µg/ml.

The incubation may be for about 2-12 hours, possibly 4-8 hours, typically for about 5-7 hours. The incubation may be for about 6 hours. The incubation may be for at least 6 hours. The incubation may be for 6 hours.

Addition of an anti-coagulant to the incubation medium may improve the yield of the cell-preparation. The anti-coagulant in the incubation medium may be of the same concentration as within the freezing medium. The incubation medium may comprise an anti-coagulant selected from: heparin, ACD Formula A or a combination thereof. The anti-coagulant used in the incubation medium may be ACD Formula A containing heparin at a concentration of 10 U/ml.

The incubation medium may comprise heparin. The heparin in the incubation medium may be at a concentration of between 0.1-2.5 U/ml. The heparin in the incubation medium may be at a concentration of between 0.1-2.5 U/ml, possibly between 0.3-0.7 U/ml, typically about 0.5 U/ml. The heparin in the incubation medium may be at a concentration of about 0.5 U/ml.

The incubation medium may comprise ACD Formula A. The ACD Formula A in the incubation medium may be at a concentration of between 1%-15% v/v. The ACD Formula A in the incubation medium may be at a concentration of between 1%-15% v/v, possibly between 4%-7% v/v, typically about 5% v/v. The ACD Formula A in the incubation medium may be at a concentration of about 5% v/v.

Improvement in the yield of the cell-preparation may comprise improvement in the number of the early-apoptotic viable cells of the preparation out of the number of frozen cells from which the preparation was produced.

Addition of an anti-coagulant to the freezing medium may contribute to a high and stable yield between different preparations of the pharmaceutical population. Addition of an anti-coagulant at least to the freezing medium and incubation medium may result in a high and stable yield between different preparations of the pharmaceutical composition, regardless to the cell collection protocol used.

The freezing medium may comprise an anti-coagulant selected from heparin, ACD Formula A or a combination thereof. The anti-coagulant used in the freezing medium may be ACD Formula A containing heparin at a concentration of 10 U/ml. The freezing medium may comprise 5% v/v of ACD Formula A solution comprising heparin at a concentration of 10 U/ml.

The freezing medium may comprise heparin. The heparin in the freezing medium may be at a concentration of between 0.1-2.5 U/ml. The heparin in the freezing medium may be at a concentration of between 0.1-2.5 U/ml, possibly between 0.3-0.7 U/ml, typically about 0.5 U/ml. The heparin in the freezing medium may be at a concentration of about 0.5 U/ml.

The freezing medium may comprise ACD Formula A. The ACD Formula A in the freezing medium may be at a concentration of between 1%-15% v/v. The ACD Formula A in the freezing medium is at a concentration of between 1%-15% v/v, possibly between 4%-7% v/v, typically about 5% v/v. The ACD Formula A in the freezing medium may be at a concentration of about 5% v/v.

Addition of an anti-coagulant to the incubation medium and/or freezing medium may result in a high and stable cell yield within the population regardless of the triglyceride level in the blood of the donor. Addition of an anti-coagulant to the incubation medium and/or freezing medium may result in a high and stable cell yield within the composition the invention when obtained from the blood of a donor having normal or high triglyceride level. Addition of an anti-coagulant at least to the incubation medium, may result in a high and stable cell yield within the composition regardless of the triglyceride level in the blood of the donor. Addition of an anti-coagulant to the freezing medium and incubation medium may result in a high and stable cell yield within the composition regardless of the triglyceride level in the blood of the donor.

The freezing medium and/or incubation medium and/or washing medium may comprise heparin at a concentration of at least 0.1 U/ml, possibly at least 0.3 U/ml, typically at least 0.5 U/ml. The freezing medium and/or incubation medium and/or washing medium may comprise ACD Formula A at a concentration of at least 1% v/v, possibly at least 3% v/v, typically at least 5% v/v.

The mononuclear-enriched cell composition may undergo at least one washing step following cell collection and prior to being re-suspended in the freezing medium and frozen. The mononuclear-enriched cell composition may undergo at least one washing step following freezing and thawing. Washing steps may comprise centrifugation of the mononuclear-enriched cell composition followed by supernatant extraction and re-suspension in washing medium.

The mononuclear-enriched cell composition may undergo at least one washing step between each stage of the production of an early apoptotic cell population. Anti-coagulant may be added to washing media during washing steps throughout the production of an early apoptotic cell population. The mononuclear-enriched cell composition may undergo at least one washing step following incubation. The mononuclear-enriched cell composition may undergo at least one washing step following incubation using PBS. In some instances, anti-coagulant is not added to the final washing step prior to re-suspension of the cell-preparation in the administration medium. In some instances, anti-coagulant is not added to the PBS used in the final washing step prior to re-suspension of the cell-preparation in the administration medium. In certain instances, anti-coagulant is not added to the administration medium.

The cell concentration during incubating may be about 5x10⁶ cells/ml.

The mononuclear-enriched cell composition may be suspended in an administration medium following freezing, thawing and incubating, thereby resulting in the pharmaceutical population. The administration medium may comprise a suitable physiological buffer. Examples of a suitable physiological buffer may be saline solution, Phoshpate Buffered Saline (PBS), Hank's Balanced Salt Solution (HBSS), and the like. The administration medium may comprise PBS. The administration medium may comprise supplements conducive to maintaining the viability of the cells. The mononuclear-enriched cell composition may be filtered prior to administration. The mononuclear-enriched cell composition may be filtered prior to administration using a filter of at least 200µm.

The mononuclear-enriched cell population may be re-suspended in an administration medium such that the final volume of the resulting cell-preparation is between 100-1000ml, possibly between 200-800ml, typically between 300-600ml.

Cell collection may refer to obtaining a mononuclear-enriched cell composition. Washing steps performed during the production of an early apoptotic cell population may be performed in a washing medium. Washing steps performed up until the incubation step of the production of an early apoptotic cell population may be performed in a washing medium. The washing medium may comprise RPMI 1640 medium supplemented with L-glutamine and Hepes. The washing medium may comprise RPMI 1640 medium supplemented with 2 mM L-glutamine and 10 mM Hepes.

The washing medium may comprise an anti-coagulant. The washing medium may comprise an anti-coagulant selected from heparin, ACD Formula A or a combination thereof. The concentration of the anti-coagulant in the washing medium may be the same concentration as in the freezing medium. The concentration of the anti-coagulant in the washing medium may be the same concentration as in the incubation medium. The anti-coagulant used in the washing medium may be ACD Formula A containing heparin at a concentration of 10 U/ml.

The washing medium may comprise heparin. The heparin in the washing medium may be at a concentration of between 0.1-2.5 U/ml. The heparin in the washing medium may be at a concentration of between 0.1-2.5 U/ml, possibly between 0.3-0.7 U/ml, typically about 0.5 U/ml. The heparin in the washing medium may be at a concentration of about 0.5 U/ml.

The washing medium may comprise ACD Formula A. The ACD Formula A in the washing medium may be at a concentration of between 1%-15% v/v, possibly between 4%-7% v/v, typically about 5% v/v. The ACD Formula A in the washing medium may be at a concentration of about 5% v/v.

The mononuclear-enriched cell composition may be thawed several hours prior to the intended administration of the population to a subject. The mononuclear-enriched cell composition may be thawed at about 33°C-39°C. The mononuclear-enriched cell composition may be thawed for about 30-240 seconds, preferably 40-180 seconds, most preferably 50-120 seconds.

The mononuclear-enriched cell composition may be thawed at least 10 hours prior to the intended administration of the population, alternatively at least 20, 30, 40 or 50 hours prior to the intended administration of the population. The mononuclear-enriched cell composition may be thawed at least 15-24 hours prior to the intended administration of the population. The mononuclear-enriched cell composition may be thawed at least about 24 hours prior to the intended administration of the population, or at least 20 hours prior to the intended administration of the population, or 30 hours prior to the intended administration of the population, or at least 24 hours prior to the intended administration of the population. The mononuclear-enriched cell composition may undergo at least one step of washing in the washing medium before and/or after thawing.

The composition may further comprise methylprednisolone. In some cases, the concentration of methylprednisolone does not exceed 30µg/ml.

The apoptotic cells may be used at a high dose. The apoptotic cells may be used at a high concentration. Human apoptotic polymorphonuclear neutrophils (PMNs) may be used. A group of cells, of which 50% are apoptotic cells, may be used. Apoptotic cells may be verified by May-Giemsa-stained cytopreps. Viability of cells may be assessed by trypan blue exclusion. The apoptotic and necrotic status of the cells may be confirmed by annexin V/propidium iodide staining with detection by FACS.

Apoptotic cells disclosed herein may comprise no necrotic cells. Apoptotic cells disclosed herein may comprise less than 1% necrotic cells, may comprise less than 2% necrotic cells, may comprise less than 3% necrotic cells, may comprise less than 4% necrotic cells, or may comprise less than 5% necrotic cells.

The apoptotic cells may be prepared from cells obtained from a subject other than the subject that will receive said apoptotic cells. In some instances, the invention as disclosed herein may comprise an additional step that is useful in overcoming rejection of allogeneic donor cells, including one or more steps described in U.S. Patent Application Publication 20130156794. This may comprise the step of full or partial lymphodepletion prior to administration of the apoptotic cells, e.g. allogeneic apoptotic cells. The lymphodepletion may be adjusted so that it delays the host versus graft reaction for a period sufficient to allow the allogeneic apoptotic cells to control cytokine release. This may comprise the step of administering agents that delay egression of the allogeneic apoptotic T-cells from lymph nodes, such as 2-amino-2-[2-(4-octylphenyl)ethyl]propane-1,3-diol (FTY720), 5-[4-phenyl-5-(trifluoromethyl)thiophen-2-yl]-3-[3-(trifluoromethyl)pheny-1]1,2,4-oxadiazole (SEW2871), 3-(2-(-hexylphenylamino)-2-oxoethylamino)propanoic acid (W123), 2-ammonio-4-(2-chloro-4-(3-phenoxyphenylthio)phenyl)-2-(hydroxymethyl)but-yl hydrogen phosphate (KRP-203 phosphate) or other agents known in the art, may be used as part of the compositions to allow the use of allogeneic apoptotic cells having efficacy and lacking initiation of graft vs host disease. In another possibility, MHC expression by the allogeneic apoptotic T-cells is silenced to reduce the rejection of the allogeneic cells.

Producing a population of mononuclear apoptotic cell comprising a decreased percent of non-quiescent non-apoptotic viable cells; a suppressed cellular activation of any living non-apoptotic cells; or a reduced proliferation of any living non-apoptotic cells; or any combination thereof, may comprise the following steps, obtaining a mononuclear-enriched cell population of peripheral blood; freezing said mononuclear-enriched cell population in a freezing medium comprising an anticoagulant; thawing said mononuclear-enriched cell population; incubating said mononuclear-enriched cell population in an apoptosis inducing incubation medium comprising methylprednisolone at a final concentration of about 10-100 µg/mL and an anticoagulant; resuspending said apoptotic cell population in an administration medium; and inactivating said mononuclear-enriched population, wherein said inactivation occurs following apoptotic induction, thereby producing a population of mononuclear apoptotic cell comprising a decreased percent of non-quiescent non-apoptotic cells; a suppressed cellular activation of any living non-apoptotic cells; or a reduced proliferation of any living non-apoptotic cells; or any combination thereof.

The early apoptotic mononuclear enriched cell population for use in this invention is irradiated. This may comprise the step of irradiating a population of apoptotic cells derived from a subject prior to administration of the population of apoptotic cells to the same subject (autologous ApoCells; Autocetra). In some embodiments, apoptotic cells derived from a subject may be irradiated prior to administration of the population of apoptotic cells to a recipient (allogeneic ApoCells; Allocetra).

The cells may be irradiated in a way that will decrease proliferation and/or activation of residual viable cells within the apoptotic cell population. The cells may be irradiated in a way that reduces the percent of viable non-apoptotic cells in a population. The percent of viable non-apoptotic cells in an inactivated early apoptotic cell population may be reduced to less than 50% of the population, reduced to less than 40% of the population, reduced to less than 30% of the population, reduced to less than 20% of the population, reduced to less than 10% of the population, or reduced to 0% of the population.

The irradiated apoptotic cells for use according to the present invention may preserve all their early apoptotic-, immune modulation-, stability-properties. The irradiation step may use UV radiation. The radiation step may use gamma radiation. The apoptotic cells may comprise a decreased percent of living non-apoptotic cells, comprise a preparation having a suppressed cellular activation of any living non-apoptotic cells present within the apoptotic cell preparation, or may comprise a preparation having reduced proliferation of any living non-apoptotic cells present within the apoptotic cell preparation, or any combination thereof.

In some embodiments, irradiation of apoptotic cells does not increase the population of dead cells (PI+) compared with apoptotic cells not irradiated. In some embodiments, irradiation of apoptotic cells does not increase the population of dead cells (PI+) by more than about 1% compared with apoptotic cells not irradiated. In some embodiments, irradiation of apoptotic cells does not increase the population of dead cells (PI+) by more than about 2% compared with apoptotic cells not irradiated. In some embodiments, irradiation of apoptotic cells does not increase the population of dead cells (PI+) by more than about 3% compared with apoptotic cells not irradiated. In some embodiments, irradiation of apoptotic cells does not increase the population of dead cells (PI+) by more than about 4% compared with apoptotic cells not irradiated. In some embodiments, irradiation of apoptotic cells does not increase the population of dead cells (PI+) by more than about 5% compared with apoptotic cells not irradiated. In some embodiments, irradiation of apoptotic cells does not increase the population of dead cells (PI+) by more than about 6% compared with apoptotic cells not irradiated. In some embodiments, irradiation of apoptotic cells does not increase the population of dead cells (PI+) by more than about 7% compared with apoptotic cells not irradiated. In some embodiments, irradiation of apoptotic cells does not increase the population of dead cells (PI+) by more than about 8% compared with apoptotic cells not irradiated. In some embodiments, irradiation of apoptotic cells does not increase the population of dead cells (PI+) by more than about 9% compared with apoptotic cells not irradiated. In some embodiments, irradiation of apoptotic cells does not increase the population of dead cells (PI+) by more than about 10% compared with apoptotic cells not irradiated. In some embodiments, irradiation of apoptotic cells does not increase the population of dead cells (PI+) by more than about 15% compared with apoptotic cells not irradiated. In some embodiments, irradiation of apoptotic cells does not increase the population of dead cells (PI+) by more than about 20%, 25%, 30%, 35%, 40%, 45%, or 50% compared with apoptotic cells not irradiated.

A cell population comprising a reduced or non-existent fraction of living non-apoptotic cells may provide a mononuclear early apoptotic cell population that does not have any living / viable cells. A cell population comprising a reduced or non-existent fraction of living non-apoptotic cells may provide a mononuclear apoptotic cell population that does not elicit GVHD in a recipient.

Use of irradiated ApoCells may remove the possible graft versus leukemia effect use of an apoptotic population (that includes a minor portion of viable cells) may cause, demonstrating that the effects shown here in the Examples (See Example 3) result from the apoptotic cells and not from a viable proliferating population of cells with cellular activity, present within the apoptotic cell population.

Apoptotic cells derived from WBCs from a donor are irradiated prior to administration to a recipient. In some embodiments, cells are irradiated in a way that will avoid proliferation and/or activation of residual viable cells within the apoptotic cell population. In another embodiment, the irradiated apoptotic cells preserve all their early apoptotic-, immune modulation- , stability-properties. In another embodiment, the irradiation step uses UV radiation. In another embodiment, the radiation step uses gamma radiation. The apoptotic cells may comprise a decreased percent of living non-apoptotic cells, may comprise a preparation having a suppressed cellular activation of any living non-apoptotic cells present within the apoptotic cell preparation, or may comprise a preparation having reduced proliferation of any living non-apoptotic cells present within the apoptotic cell preparation, or any combination thereof.

Apoptotic cells may comprise a pooled mononuclear apoptotic cell preparation. A pooled mononuclear apoptotic cell preparation may comprise mononuclear cells in an early apoptotic state, wherein said pooled mononuclear apoptotic cells comprise a decreased percent of living non-apoptotic cells, a preparation having a suppressed cellular activation of any living non-apoptotic cells, or a preparation having reduced proliferation of any living non-apoptotic cells, or any combination thereof. The pooled mononuclear apoptotic cells have been irradiated. The pooled mononuclear apoptotic cell preparation may originate from the white blood cell fraction (WBC) obtained from donated blood.

The apoptotic cell preparation is irradiated. In another embodiment, said irradiation comprises gamma irradiation or UV irradiation. In yet another embodiment, the irradiated preparation has a reduced number of non-apoptotic cells compared with a non-irradiated apoptotic cell preparation. The irradiated preparation may have a reduced number of proliferating cells compared with a non-irradiated apoptotic cell preparation. The irradiated preparation may have a reduced number of potentially immunologically active cells compared with a non-irradiated apoptotic cell population.

Pooled blood may comprise 3rd party blood not matched between donor and recipient (allogeneic).

A skilled artisan would appreciate that the term "pooled" may encompass blood collected from multiple donors, prepared and possibly stored for later use. This combined pool of blood may then be processed to produce a pooled mononuclear apoptotic cell preparation. A pooled mononuclear apoptotic cell preparation may ensure that a readily available supply of mononuclear apoptotic cells is available. Cells may be pooled just prior to the incubation step wherein apoptosis is induced. Cells may be pooled following the incubation step at the step of resuspension. Cells may be pooled just prior to an irradiation step. Cells may be pooled following an irradiation step. Cells may be pooled at any step in the methods of preparation.

A population of pooled mononuclear apoptotic cells may comprise blood or apoptotic cells or cells from any step within the process of making early apoptotic cells that have been pooled. Cells may be allogeneic. Cells may be autologous.

A pooled apoptotic cell preparation may be derived from cells present in between about 2 and 25 units of blood. The pooled apoptotic cell preparation may be comprised of cells present in between about 2-5, 2-10, 2-15, 2-20, 5-10, 5-15, 5-20, 5-25, 10-15, 10-20, 10-25, 6-13, or 6-25 units of blood. The pooled apoptotic cell preparation may be comprised of cells present in about 2, 3, 4, 5, 6, 7, 8, 9, 10, 11, 12, 13, 14, 15, 16, 17, 18, 19, 20, 21, 22, 23, 24 or 25 units of blood. The number of units of blood needed is also dependent upon the efficiency of WBC recovery from blood. For example, low efficiency WBC recovery would lead to the need for additional units, while high efficiency WBC recovery would lead to fewer units needed. Each unit may be a bag of blood. A pooled apoptotic cell preparation may be comprised of cells present in at least 25 units of blood, at least 50 units of blood, or at least 100 units of blood.

The units of blood may comprise white blood cell (WBC) fractions from blood donations. The donations may be from a blood center or blood bank. The donations may be from donors in a hospital gathered at the time of preparation of the pooled apoptotic cell preparation. Units of blood comprising WBCs from multiple donors may be saved and maintained in an independent blood bank created for the purpose of compositions as disclosed herein. A blood bank developed for the purpose of compositions disclosed herein, may be able to supply units of blood comprising WBC from multiple donors and comprises a leukapheresis unit.

The units of pooled WBCs are not restricted by HLA matching. Therefore, the resultant pooled apoptotic cell preparation may comprise cell populations not restricted by HLA matching. Accordingly, a pooled mononuclear apoptotic cell preparation may comprise allogeneic cells.

An advantage of a pooled mononuclear apoptotic cell preparation that may be derived from pooled WBCs not restricted by HLA matching, is a readily available source of WBCs and reduced costs of obtaining WBCs.

Pooled blood may comprise blood from multiple donors independent of HLA matching. Pooled blood may comprise blood from multiple donors wherein HLA matching with the recipient has been taken into consideration. For example, wherein 1 HLA allele, 2 HLA alleles, 3 HLA alleles, 4 HLA alleles, 5 HLA alleles, 6 HLA alleles, or 7 HLA alleles may have been matched between donors and recipient. Multiple donors may be partially matched, for example some of the donors have been HLA matched wherein 1 HLA allele, 2 HLA alleles, 3 HLA alleles, 4 HLA alleles, 5 HLA alleles, 6 HLA alleles, or 7 HLA alleles may have been matched between some of the donors and recipient.

Some viable non-apoptotic cells (apoptosis resistant) may remain following the induction of apoptosis step described below (Example 1). The presence of these viable non-apoptotic cells may be observed prior to an irradiation step. These viable non-apoptotic cells may be able to proliferate or be activated. The pooled mononuclear apoptotic cell preparation derived from multiple donors may be activated against the host, activated against one another, or both.

An irradiated cell preparation as disclosed herein may have suppressed cellular activation and reduced proliferation compared with a non-irradiated cell preparation. In another embodiment, the irradiation comprises gamma irradiation or UV irradiation. In another embodiment, an irradiated cell preparation has a reduced number of non-apoptotic cells compared with a non-irradiated cell preparation. In some embodiments, the irradiation comprises about 10 Gray units (Gy) to 60 Gy. In some embodiments, the irradiation comprises about 10 Gray units (Gy) to 6 Gy. In some embodiments, the irradiation comprises about 10 Gray units (Gy) to 50 Gy. In some embodiments, the irradiation comprises about 10 Gray units (Gy) to 40 Gy. In some embodiments, the irradiation comprises about 10 Gray units (Gy) to 30 Gy. In some embodiments, the irradiation comprises about 10 Gray units (Gy) to 20 Gy. In some embodiments, the irradiation comprises about 10 Gray units (Gy) to 15 Gy.

In another embodiment, the irradiation comprises about 10 Gray units (Gy). In another embodiment, the irradiation comprises about 15 Gray units (Gy). In another embodiment, the irradiation comprises about 20 Gray units (Gy). In another embodiment, the irradiation comprises about 25 Gray units (Gy). In another embodiment, the irradiation comprises about 30 Gray units (Gy). In another embodiment, the irradiation comprises about 35 Gray units (Gy). In another embodiment, the irradiation comprises about 40 Gray units (Gy). In another embodiment, the irradiation comprises about 45 Gray units (Gy). In another embodiment, the irradiation comprises about 50 Gray units (Gy). In another embodiment, the irradiation comprises about 55 Gray units (Gy). In another embodiment, the irradiation comprises about 60 Gray units (Gy). Gray. In another embodiment, an irradiated pooled apoptotic cell preparation maintains the same or a similar apoptotic profile, stability and efficacy as a non-irradiated pooled apoptotic cell preparation.

A pooled mononuclear apoptotic cell preparation may be stable for up to 24 hours. A pooled mononuclear apoptotic cell preparation may be stable for at least 24 hours, may be stable for more than 24 hours, may be stable for up to 36 hours, may be stable for at least 36 hours, may be stable for more than 36 hours, may be stable for up to 48 hours, may be stable for at least 48 hours, or may be stable for more than 48 hours.

Methods of producing the pooled cell preparation comprising an irradiation step may preserve the early apoptotic, immune modulation, and stability properties observed in an apoptotic preparation derived from a single match donor A pooled mononuclear apoptotic cell preparation does not elicit a graft versus host disease (GVHD) response.

Irradiation of the cell preparation is considered safe in the art. Irradiation procedures are currently performed on a routine basis to donated blood to prevent reactions to WBC.

The percent of apoptotic cells in a pooled mononuclear apoptotic cell preparation as disclosed herein may be close to 100%, thereby reducing the fraction of living non-apoptotic cells in the cell preparation. The percent of apoptotic cells may be at least 40%, the percent of apoptotic cells may be at least 50%, the percent of apoptotic cells may be at least 60%, the percent of apoptotic cells may be at least 70%, the percent of apoptotic cells may be at least 80%, the percent of apoptotic cells may be at least 90%, or the percent of apoptotic cells may be at least 99%. Accordingly, a cell preparation comprising a reduced or non-existent fraction of living non-apoptotic cells may provide a pooled mononuclear apoptotic cell preparation that does not elicit GVHD in a recipient.

Alternatively, the percentage of living non-apoptotic WBC may be reduced by specifically removing the living cell population, for example by targeted precipitation. The percent of living non-apoptotic cells may be reduced using magnetic beads that bind to phosphatidylserine. The percent of living non-apoptotic cells may be reduced using magnetic beads that bind a marker on the cell surface of non-apoptotic cells but not apoptotic cells. The apoptotic cells may be selected for further preparation using magnetic beads that bind to a marker on the cell surface of apoptotic cells but not non-apoptotic cells. The percentage of living non-apoptotic WBC may be reduced by the use of ultrasound.

The apoptotic cells may be from pooled third-party donors.

A pooled cell preparation may comprise at least one cell type selected from: lymphocytes, monocytes or natural killer cells. A pooled cell preparation may comprise an enriched population of mononuclear cells. A pooled mononuclear may be a mononuclear enriched cell preparation comprises cell types selected from lymphocytes, monocytes and natural killer cells. The mononuclear enriched cell preparation may comprise no more than 15%, alternatively no more than 10%, typically no more than 5% polymorphonuclear leukocytes, also known as granulocytes (i.e., neutrophils, basophils and eosinophils). A pooled mononuclear cell preparation may be devoid of granulocytes.

The pooled mononuclear enriched cell preparation may comprise no more than 15%, alternatively no more than 10%, typically no more than 5% CD15high expressing cells. The pooled apoptotic cell preparation may comprise less than 15% CD15 high expressing cells.

The pooled mononuclear enriched cell preparation may comprise at least 80% mononuclear cells, at least 85% mononuclear cells, alternatively at least 90% mononuclear cells, or at least 95% mononuclear cells. According to some instances, the pooled mononuclear enriched cell preparation comprises at least 85% mononuclear cells.

Any pooled cell preparation that has a final pooled percent of mononuclear cells of at least 80% may be considered a pooled mononuclear enriched cell preparation. Thus, pooling cell preparations having increased polymorphonuclear cells (PMN) with cell preparations having high mononuclear cells with a resultant "pool" of at least 80% mononuclear cells may comprise a preparation. Mononuclear cells may comprise lymphocytes and monocytes.

A skilled artisan would appreciate that the term "mononuclear cells" may encompass leukocytes having a one lobed nucleus. A pooled apoptotic cell preparation may comprise less than 5% polymorphonuclear leukocytes.

The apoptotic cells may be T-cells. The apoptotic cells may be derived from the same pooled third-party donor T-cells as the CAR T-cells. The apoptotic cells may be derived from the CAR T-cell population.

The "apoptotic cells" may be cells chosen from any cell type of a subject, or any commercially available cell line, subjected to a method of inducing apoptosis known to the person skilled in the art. The method of inducing apoptosis may be hypoxia, ozone, heat, radiation, chemicals, osmotic pressure, pH shift, X-ray irradiation, gamma- ray irradiation, UV irradiation, serum deprivation, corticoids or combinations thereof, or any other method described herein or known in the art. The method of inducing apoptosis may produce apoptotic cells in an early apoptotic state.

The apoptotic cells may be leukocytes.

The apoptotic leukocytes may be derived from peripheral blood mononuclear cells (PBMC). The leukocytes may be from pooled third-party donors. The leukocytes may be allogeneic.

According to some instances, the apoptotic cells may be provided by selecting non-adherent leukocytes and submitting them to apoptosis induction, followed by a cell culture step in culture medium. "Leukocytes" may be derived from any lineage, or sub-lineage, of nucleated cells of the immune system and/or hematopoietic system, including dendritic cells, macrophages, mast-cells, basophils, hematopoietic stem cells, bone marrow cells, natural killer cells, and the like. The leukocytes may be derived or obtained in any of various suitable ways, from any of various suitable anatomical compartments, according to any of various commonly practiced methods, depending on the application and purpose, desired leukocyte lineage, etc. The source leukocytes may be primary leukocytes. The source leukocytes may be primary peripheral blood leukocytes.

Primary lymphocytes and monocytes may be conveniently derived from peripheral blood. Peripheral blood leukocytes include 70-95 percent lymphocytes, and 5-25 percent monocytes.

Methods for obtaining specific types of source leukocytes from blood are routinely practiced. Obtaining source lymphocytes and/or monocytes can be achieved, for example, by harvesting blood in the presence of an anticoagulant, such as heparin or citrate. The harvested blood is then centrifuged over a Ficoll cushion to isolate lymphocytes and monocytes at the gradient interface, and neutrophils and erythrocytes in the pellet.

Leukocytes may be separated from each other via standard immunomagnetic selection or immunofluorescent flow cytometry techniques according to their specific surface markers, or via centrifugal elutriation. For example, monocytes can be selected as the CD14⁺ fraction, T-lymphocytes can be selected as CD3⁺ fraction, B-lymphocytes can be selected as the CD19⁺ fraction, macrophages as the CD206⁺ fraction.

Lymphocytes and monocytes may be isolated from each other by subjecting these cells to substrate-adherent conditions, such as by static culture in a tissue culture-treated culturing recipient, which results in selective adherence of the monocytes, but not of the lymphocytes, to the cell-adherent substrate.

Leukocytes may also be obtained from peripheral blood mononuclear cells (PBMCs), which may be isolated as described herein.

One of ordinary skill in the art will possess the necessary expertise to suitably culture primary leukocytes so as to generate desired quantities of cultured source leukocytes as disclosed herein, and ample guidance for practicing such culturing methods is available in the literature of the art.

One of ordinary skill in the art will further possess the necessary expertise to establish, purchase, or otherwise obtain suitable established leukocyte cell lines from which to derive the apoptotic leukocytes. Suitable leukocyte cell lines may be obtained from commercial suppliers, such as the American Tissue Type Collection (ATCC). It will be evident to the person skilled in the art that source leukocytes should not be obtained via a technique which will significantly interfere with their capacity to produce the apoptotic leukocytes.

The apoptotic cells may be apoptotic lymphocytes. Apoptosis of lymphocytes, such as primary lymphocytes, may be induced by treating the primary lymphocytes with serum deprivation, a corticosteroid, or irradiation. Inducing apoptosis of primary lymphocytes via treatment with a corticosteroid may be affected by treating the primary lymphocytes with dexamethasone. For example, with dexamethasone at a concentration of about 1 micromolar. Inducing apoptosis of primary lymphocytes via irradiation may be affected by treating the primary lymphocytes with gamma- irradiation. For example, with a dosage of about 66 rad. Such treatment results in the generation of apoptotic lymphocytes suitable for the co-culture step with phagocytes.

Apoptotic cells may be apoptotic monocytes, such as primary monocytes. To generate apoptotic monocytes the monocytes may be subjected to in vitro conditions of substrate/surface-adherence under conditions of serum deprivation. Such treatment results in the generation of non-pro-inflammatory apoptotic monocytes suitable for the co-culture step with phagocytes.

The apoptotic cells may be any apoptotic cells described herein, including allogeneic apoptotic cells, third party apoptotic cells, and pools of apoptotic cells.

### Apoptotic Cell Administration

Surprisingly, the apoptotic cells of the present invention reduce production of pro- and anti-inflammatory cytokines/chemokines. In some embodiments, administration of apoptotic cells reduces production of pro-inflammatory cytokines/chemokines including IL-1, IL-6, IL-8, IL-10, IL-1β, IL-2, IL-15, IL-22, MIP-1β, MCP-1, MDC, IP-10, fractalkine, IL-9, or TNFα, alone or in combination. In one embodiment, the apoptotic cells affect cytokine expression levels in synovial fluid. In one embodiment, the apoptotic cells affect cytokine expression levels in joint fluid.

In some embodiments, administration of apoptotic cells of the present invention inhibits one or more pro- and anti-inflammatory cytokines. In some embodiments, administration of apoptotic cells inhibits one or more pro-inflammatory cytokine. In some embodiments, administration of apoptotic cells inhibits one or more anti-inflammatory cytokines. In some embodiments, the pro- and anti-inflammatory cytokine comprises IL-1, IL-6, IL-8, IL-10, IL-1β, IL-2, IL-15, IL-22, MIP-1β, MCP-1, MDC, IP-10, fractalkine, IL-9, or TNFα, or any combination thereof. In another embodiment, administration of apoptotic cells promotes the secretion of one or more anti-inflammatory cytokines. In some embodiments, at least one cytokine or chemokine having abnormal expression or content is downregulated by administration of apoptotic cells. In some embodiments, each cytokine or chemokine having abnormal expression or content is downregulated by administration of apoptotic cells.

In some embodiments, at least one anti-inflammatory cytokine or chemokine having abnormal expression or content is downregulated by administration of apoptotic cells. In some embodiments, at least one pro-inflammatory cytokine or chemokine having abnormal expression or content is downregulated by administration of apoptotic cells. In some embodiments, a combination of pro- and anti-inflammatory cytokines or chemokines having abnormal expression or content are downregulated by administration of apoptotic cells. In some embodiments, any of IL-1, IL-6, IL-8, IL-10, IL-1β, IL-2, IL-15, IL-22, MIP-1β, MCP-1, MDC, IP-10, fractalkine, IL-9, or TNFα, or any combination thereof, having abnormal expression or content, for example in synovial fluid or joint fluid in the area of a joint are downregulated by administration of apoptotic cells. In some embodiments, any of IL-1, IL-6, IL-8, IL-10, IL-1β, IL-2, IL-15, IL-22, MIP-1β, MCP-1, MDC, IP-10, fractalkine, IL-9, or TNFα, or any combination thereof, having abnormal expression or content are downregulated by administration of apoptotic cells. (See Example 4)

In some embodiments, a dose of about 100 x 10⁶ - 210 x 10⁶ apoptotic cells is administered. In some embodiments, a dose of about 140 x 10⁶ - 210 x 10⁶ apoptotic cells is administered. In some embodiments, a dose of about 100 x 10⁶ - 140 x 10⁶ apoptotic cells is administered. In some embodiments, a dose of about 10-100 x 10⁶ apoptotic cells is administered.

In some embodiments, a dose of about 1 x 10⁶ - x 10⁹ apoptotic cells is administered. In some embodiments, a dose of about 10 x 10⁶ - x 10⁹ apoptotic cells is administered. In some embodiments, a dose of about 1 x 10⁶ - x 10⁸ apoptotic cells is administered. In some embodiments, a dose of about 1 x 10⁷ - x 10⁸ apoptotic cells is administered. In some embodiments, a dose of about 1 x 10⁶ - x 10⁷ apoptotic cells is administered. In some embodiments, a dose of about 1 x 10⁷ - x 10⁹ apoptotic cells is administered. In some embodiments, a dose of about 1 x 10⁸ - x 10⁹ apoptotic cells is administered.

In some embodiments, a dose of about 20 x 10⁶ apoptotic cells is administered. In some embodiments, a dose of about 30 x 10⁶ apoptotic cells is administered. In some embodiments, a dose of about 40 x 10⁶ apoptotic cells is administered. In some embodiments, a dose of about 50 x 10⁶ apoptotic cells is administered. In some embodiments, 60 x 10⁶ apoptotic cells is administered. In some embodiments, a dose of about 60 x 10⁶ apoptotic cells is administered. In some embodiments, a dose of about 70 x 10⁶ apoptotic cells is administered. In some embodiments, a dose of about 80 x 10⁶ apoptotic cells is administered. In some embodiments, a dose of about 90 x 10⁶ apoptotic cells is administered. In some embodiments, a dose of about 1-15 x 10⁷ apoptotic cells is administered. In some embodiments, a dose of about 10 x 10⁷ apoptotic cells is administered. In some embodiments, a dose of about 11 x 10⁷ apoptotic cells is administered. In some embodiments, a dose of about 12 x 10⁷ apoptotic cells is administered. In some embodiments, a dose of about 13 x 10⁷ apoptotic cells is administered. In some embodiments, a dose of about 14 x 10⁷ apoptotic cells is administered. In some embodiments, a dose of about 15 x 10⁷apoptotic cells is administered.

In some embodiments, a dose of 10x10⁶ apoptotic cells is administered. In another embodiment, a dose of 10x10⁷ apoptotic cells is administered. In another embodiment, a dose of 10x10⁸ apoptotic cells is administered. In another embodiment, a dose of 10x10⁹ apoptotic cells is administered. In another embodiment, a dose of 10x10¹⁰ apoptotic cells is administered. In another embodiment, a dose of 10x10¹¹ apoptotic cells is administered. In another embodiment, a dose of 10x10¹² apoptotic cells is administered. In another embodiment, a dose of 10x10⁵ apoptotic cells is administered. In another embodiment, a dose of 10x10⁴ apoptotic cells is administered. In another embodiment, a dose of 10x10³ apoptotic cells is administered. In another embodiment, a dose of 10x10² apoptotic cells is administered.

In some embodiments, a high dose of apoptotic cells is administered. In some embodiments, a dose of 35x10⁶ apoptotic cells is administered. In another embodiment, a dose of 210x10⁶ apoptotic cells is administered. In another embodiment, a dose of 70x10⁶ apoptotic cells is administered. In another embodiment, a dose of 140x10⁶ apoptotic cells is administered. In another embodiment, a dose of 35-210x10⁶ apoptotic cells is administered.

In some embodiments, a single dose of apoptotic cells is administered. In some embodiments, multiple doses of apoptotic cells are administered. In some embodiments, 2 doses of apoptotic cells are administered. In some embodiments, 3 doses of apoptotic cells are administered. In some embodiments, 4 doses of apoptotic cells are administered. In some embodiments, 5 doses of apoptotic cells are administered. In some embodiments, 6 doses of apoptotic cells are administered. In some embodiments, 7 doses of apoptotic cells are administered. In some embodiments, 8 doses of apoptotic cells are administered. In some embodiments, 9 doses of apoptotic cells are administered. In some embodiments, more than 9 doses of apoptotic cells are administered. In some embodiments, multiple doses of apoptotic cells are administered. In some embodiments, multiple doses of apoptotic cells are administered during the treatment of a subject in need.

Each of the doses described herein may in some embodiments be ± about 5 %, 10%, 15%, 20%, or 25% of the dose of apoptotic cells. In some embodiments, the dose of apoptotic cells is ± about 5 % of the dose of apoptotic cells. In some embodiments, the dose of apoptotic cells is ± about 10% of the dose of apoptotic cells. In some embodiments, the dose of apoptotic cells is ± about 15% of the dose of apoptotic cells. In some embodiments, the dose of apoptotic cells is ± about 20% of the dose of apoptotic cells. In some embodiments, the dose of apoptotic cells is ± about 25% of the dose of apoptotic cells. In some embodiments, the dose of apoptotic cells is between ± about 5 % - 25% of the dose of apoptotic cells. For example, a dose of about 100 x 10⁶ - 210 x 10⁶ ± 20%, or about 100 x 10⁶ ± about 20%, or a dose of 120 x 10⁶ ± about 20%, or a dose of 140 x 10⁶ ± about 20%, or a dose of 160 x 10⁶ ± about 20%, or a dose of 180 x 10⁶ ± about 20%, or a dose of 200 x 10⁶ ± about 20%, etc. Similar embodiments include doses ± 5, 10, 15, 20, or 25%.

In some embodiments, a dose of apoptotic cells is administered daily. In some embodiments, a dose of apoptotic cells is administered weekly. In some embodiments, a dose of apoptotic cells is administered semi-weekly (twice a week). In some embodiments, a dose of apoptotic cells is administered bi-weekly (every two weeks). In some embodiments, a dose of apoptotic cells is administered monthly. In some embodiments, a dose of apoptotic cells is administered in a non-regular regime, for example daily for a given time period followed by semi-weekly, or weekly, or bi-weekly, or monthly administration, or a combination thereof.

In some embodiments, the apoptotic cells may be administered by any route known in the art that would apply cells directly to an area of need including injection and infusion. In some embodiments, administration comprises injection and/or infusion directly into a joint. In some embodiments, administration comprises injection and/or infusion adjacent to a joint. Apoptotic cells for injection may be in the form of a pharmaceutical composition formulated as a sterile injectable solution.

In some embodiments, the apoptotic cells may be administered by topical administration, wherein cells or a pharmaceutical composition comprising the apoptotic cells are applied directly to an area of need. In some embodiments, administration comprises topical application directly at the site of a joint. In some embodiments, administration comprises topical application adjacent to a joint. Apoptotic cells for topical application may be in the form of a pharmaceutical composition formulated as a topical ointment, a cream, an oil, a patch, or a dermal patch. In some embodiments, the apoptotic cells may be administered by infiltrating cartilage in the area of need, wherein cells or a pharmaceutical composition comprising the apoptotic cells are targeted directly to an area of need. In some embodiments, administration comprises infiltrating a joint with apoptotic cells or a composition thereof. In some embodiments, administration comprises infiltrating a tissue adjacent to a joint with apoptotic cells or a composition thereof.

In some embodiments, application of apoptotic cells is for local use.

### Compositions

As used herein, the terms "composition" and pharmaceutical composition" may in some embodiments, be used interchangeably having all the same qualities and meanings. In some embodiments, disclosed herein is a pharmaceutical composition for the treatment of a condition or disease as described herein. Thus, disclosed herein is a pharmaceutical composition for use in the treatment of osteoarthritis.

In some embodiments, a pharmaceutical composition comprises an early apoptotic cell population as described in detail above.

In still another embodiment, a pharmaceutical composition for the treatment of osteoarthritis comprises an effective amount of an early apoptotic cell population and a pharmaceutically acceptable excipient. In some embodiments, a composition comprising apoptotic cells is useful in the treatment of pain caused by osteoarthritis. In some embodiments, a composition comprising apoptotic cells is useful in reducing the pain of osteoarthritis. In some embodiments, a composition comprising apoptotic cells is useful in reducing the inflammation in a joint caused by osteoarthritis. In some embodiments, a composition comprising apoptotic cells is useful in reducing the swelling in or around a joint caused by osteoarthritis. In some embodiments, a composition comprising apoptotic cells is useful in inhibiting or slowing the progressive degeneration of articular cartilage in a joint. In some embodiments, a composition comprising apoptotic cells is useful in increasing movement in a joint affected by osteoarthritis. In some embodiments, a composition comprising apoptotic cells is useful in increasing range of movement in a joint affected by osteoarthritis.

Dosages of early apoptotic cells are described in detail above. An effective amount of an early apoptotic cell population includes those dosages described above, for example, dosages of early apoptotic cells in the range of about 10 x 10⁶ cells ±20% through 1 x 10⁹ cells ±20%. In some embodiments, a composition comprises an effective amount of an early apoptotic cell population and a pharmaceutically acceptable excipient.

In this invention, the composition comprises apoptotic cells in an early apoptotic state. In some embodiments, apoptotic cells comprised in a composition are pooled third party donor cells. In some embodiments, apoptotic cells comprised in a composition are allogenic donor cells. In some embodiments, apoptotic cells comprised in a composition are autologous donor cells. In some embodiments, apoptotic cells comprised in a composition are pooled autologous donor cells. The early apoptotic mononuclear enriched cell population for use in this invention is irradiated.

A skilled artisan would appreciate that a "pharmaceutical composition" may encompass a preparation of one or more of the active ingredients described herein with other chemical components such as physiologically suitable carriers and excipients. The purpose of a pharmaceutical composition is to facilitate administration of a compound to an organism.

A skilled artisan would appreciate that the phrases "physiologically acceptable carrier", "pharmaceutically acceptable carrier", "physiologically acceptable excipient", and "pharmaceutically acceptable excipient", may be used interchangeably may encompass a carrier, excipient, or a diluent that does not cause significant irritation to an organism and does not abrogate the biological activity and properties of the administered active ingredient.

A skilled artisan would appreciate that an "excipient" may encompass an inert substance added to a pharmaceutical composition to further facilitate administration of an active ingredient. In some embodiments, excipients include calcium carbonate, calcium phosphate, various sugars and types of starch, cellulose derivatives, gelatin, vegetable oils and polyethylene glycols.

Techniques for formulation and administration of drugs are found in "Remington's Pharmaceutical Sciences," Mack Publishing Co., Easton, PA, latest edition.

In some embodiments, the composition as disclosed herein comprises a therapeutic composition. In some embodiments, the composition as disclosed herein comprises a therapeutic efficacy.

In some embodiments, a composition as disclosed herein is administered once. In another embodiment, the composition is administered twice. In another embodiment, the composition is administered three times. In another embodiment, the composition is administered four times. In another embodiment, the composition is administered at least four times. In another embodiment, the composition is administered more than four times. In another embodiment, the composition is administered multiple times.

In some embodiments, a composition as disclosed herein is administered daily, weekly, monthly, or in intervals that fits the underlying disease in a specific person. In some embodiments, a composition as disclosed herein is administered daily. In some embodiments, a composition as disclosed herein is administered semi-weekly. In some embodiments, a composition as disclosed herein is administered weekly. In some embodiments, a composition as disclosed herein is administered bi-weekly. In some embodiments, a composition as disclosed herein is administered monthly. In some embodiments, a composition as disclosed herein is administered using different regimes for example to a first administration daily that after a time period is changed to semi-weekly, or weekly, or bi-weekly administration. In some embodiments, a composition as disclosed herein is administered in intervals that fits the underlying disease, for example to osteoarthritis in a specific person.

In some embodiments, a composition is injected at the site of need, for example a joint. In some embodiments, a composition is injected near the site of need, for example adjacent to a joint. In some embodiments, a composition is injected at the site of need, for example a site of articular cartilage degeneration. In some embodiments, a composition is injected near the site of need, for example adjacent to a site of articular cartilage degeneration. In some embodiments, a composition is injected at the site of need, for example a site of bone erosion. In some embodiments, a composition is injected near the site of need, for example adjacent to a site of bone erosion.

In some embodiments, a composition is infused at the site of need, for example a joint. In some embodiments, a composition is infused near the site of need, for example adjacent to a joint. In some embodiments, a composition is infused at the site of need, for example a site of articular cartilage degeneration. In some embodiments, a composition is infused near the site of need, for example adjacent to a site of articular cartilage degeneration. In some embodiments, a composition is infused at the site of need, for example a site of bone erosion. In some embodiments, a composition is infused near the site of need, for example adjacent to a site of bone erosion.

In some embodiments, a composition is infiltrated at the site of need, for example a joint. In some embodiments, the cartilage in a joint is infiltrated with apoptotic cells or a composition thereof, at the site of need. In some embodiments, a composition is infiltrated near the site of need, for example adjacent to a joint. In some embodiments, a composition is infiltrated at the site of need, for example a site of articular cartilage degeneration. In some embodiments, a composition is infiltrated near the site of need, for example adjacent to a site of articular cartilage degeneration. In some embodiments, a composition is infiltrated at the site of need, for example a site of bone erosion. In some embodiments, a composition infiltrated near the site of need, for example adjacent to a site of bone erosion.

In some embodiments, administration of compositions described herein reduces pro- and anti-inflammatory cytokine or chemokine release from synovium, or cartilage or from any joint component, or a combination thereof. In some embodiments, administration of compositions described herein reduces pro- inflammatory cytokine or chemokine release from synovium, or cartilage or from any joint component, or a combination thereof. In some embodiments, administration of compositions described herein reduces anti-inflammatory cytokine or chemokine release from synovium, or cartilage or from any joint component, or a combination thereof. In some embodiments, administration of compositions described herein rebalances the immune response in a joint. In some embodiments, administration of compositions described herein rebalances the immune response in synovial fluid present in a joint. In some embodiments, administration of compositions described herein rebalances the immune response in joint fluid. In some embodiments, administration of compositions described herein rebalances the immune response in cartilage within a joint.

### Formulations

Pharmaceutical compositions comprising early apoptotic cell populations can be conveniently provided as sterile liquid preparations, e.g., isotonic aqueous solutions, suspensions, emulsions, dispersions, or viscous compositions, which may be buffered to a selected pH, Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol, and the like) and suitable mixtures thereof.

In some embodiments, a composition comprising early apoptotic cells is comprised in a Ringer's lactate solution. In some embodiments, a composition of early apoptotic cells comprises known buffer solutions, for example normal saline or PBS.

Sterile injectable solutions can be prepared by incorporating the early apoptotic cell population in the required amount of the appropriate solvent with various amounts of the other ingredients, as desired. Such compositions may be in admixture with a suitable carrier, diluent, or excipient such as sterile water, physiological saline, glucose, dextrose, or the like. The compositions can contain auxiliary substances such as wetting, dispersing, or emulsifying agents (e.g., methylcellulose), pH buffering agents, gelling or viscosity enhancing additives, preservatives, flavoring agents, colors, and the like, depending upon the route of administration and the preparation desired. Standard texts, such as "REMINGTON'S PHARMACEUTICAL SCIENCE", 17th edition, 1985may be consulted to prepare suitable preparations, without undue experimentation.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin. Any vehicle, diluent, or additive used would have to be compatible with the genetically modified immunoresponsive cells or their progenitors.

The compositions or formulations can be isotonic, i.e., they can have the same osmotic pressure as blood and lacrimal fluid. The desired isotonicity of the compositions may be accomplished using sodium chloride, or other pharmaceutically acceptable agents such as dextrose, boric acid, sodium tartrate, propylene glycol or other inorganic or organic solutes. Sodium chloride may be preferred particularly for buffers containing sodium ions.

Viscosity of the compositions, if desired, can be maintained at the selected level using a pharmaceutically acceptable thickening agent. Methylcellulose may be preferred because it is readily and economically available and is easy to work with.

Other suitable thickening agents include, for example, xanthan gum, carboxymethyl cellulose, hydroxypropyl cellulose, carbomer, and the like. The preferred concentration of the thickener will depend upon the agent selected. The important point is to use an amount that will achieve the selected viscosity. Obviously, the choice of suitable carriers and other additives will depend on the exact route of administration and the nature of the particular dosage form, e.g., liquid dosage form (e.g., whether the composition is to be formulated into a solution, a suspension, gel or another liquid form, such as a time release form or liquid-filled form).

Those skilled in the art will recognize that the components of the compositions or formulations should be selected to be chemically inert and will not affect the viability or efficacy of the early apoptotic cell populations for use as disclosed herein. This will present no problem to those skilled in chemical and pharmaceutical principles, or problems can be readily avoided by reference to standard texts or by simple experiments (not involving undue experimentation), from this disclosure and the documents cited herein.

One consideration concerning the therapeutic use of early apoptotic cells disclosed herein is the quantity of cells necessary to achieve an optimal effect. The quantity of cells to be administered may in some embodiments, vary for the subject being treated. In addition, for local administration to a joint, the size of the joint may in some embodiments, be taken into consideration.

In some embodiments, between 10⁵ to 10¹⁰, between 10⁶ to 10⁹, or between 10⁶ and 10⁸ early apoptotic cells are administered to a human subject. The number of apoptotic cells for administration for administration may in some embodiments, comprise a range.

In some embodiments, between 1 x 10⁶ - 1 x 10⁹ 20%. In some embodiments, between 1 x 10⁶ - 1 x 10⁸ 20%. In some embodiments, between 1 x 10⁶ - 1 x 10⁷ 20%. In some embodiments, between 1 x 10⁷ - 1 x 10⁹ 20%. In some embodiments, between 1 x 10⁸ - 1 x 10⁹ 20%. In some embodiments, between 10 x 10⁶ - 500 x 10⁶ ± 20%. In some embodiments, between 10 x 10⁶ - 210 x 10⁶ ± 20%, or about 10 x 10⁶ ± about 20%, or a dose of 12 x 10⁶ ± about 20% or a dose collected from 12 x 10⁶ ± about 20% cells, or a dose of 14 x 10⁶ ± about 20% or a dose collected from 12 x 10⁶ ± about 20% cells, or a dose of 16 x 10⁶ ± about 20% or a dose collected from 16 x 10⁶ ± about 20% cells, or a dose of 18 x 10⁶ ± about 20% or a dose collected from 18 x 10⁶ ± about 20% cells, or a dose of 20 x 10⁶ ± about 20% or a dose collected from 20 x 10⁶ ± about 20% cells. In some embodiments, between 100 x 10⁶ - 210 x 10⁶ ± 20%, or about 100 x 10⁶ ± about 20%, or a dose of 120 x 10⁶ ± about 20% or a dose collected from 120 x 10⁶ ± about 20% cells, or a dose of 140 x 10⁶ ± about 20% or a dose collected from 140 x 10⁶ ± about 20% cells, or a dose of 160 x 10⁶ ± about 20% or a dose collected from 160 x 10⁶ ± about 20% cells, or a dose of 180 x 10⁶ ± about 20% or a dose collected from 180 x 10⁶ ± about 20% cells, or a dose of 200 x 10⁶ ± about 20% or a dose collected from 200 x 10⁶ ± about 20% cells, or a dose of 1 x 10⁶ ± about 20% or a dose collected from 1 x 10⁶ ± about 20% cells, or a dose of 1 x 10⁷ ± about 20% or a dose collected from 1 x 10⁷ ± about 20% cells, or a dose of 1 x 10⁸ ± about 20% or a dose collected from 1 x 10⁸ ± about 20% cells, or a dose of 1 x 10⁹ ± about 20% or a dose collected from 1 x 10⁹ ± about 20% cells.

In some embodiments, between 1 x 10⁶ - 1 x 10⁹ 5%. In some embodiments, between 1 x 10⁶ - 1 x 10⁸ 5%. In some embodiments, between 1 x 10⁶ - 1 x 10⁷ 5%. In some embodiments, between 1 x 10⁷ - 1 X 10⁹ 5%. In some embodiments, between 1 x 10⁸ - 1 x 10⁹ 5%. In some embodiments, between 10 x 10⁶ - 500 x 10⁶ ± 5%. In some embodiments, between 10 x 10⁶ - 210 x 10⁶ ± 5%, or about 10 x 10⁶ ± about 5%, or a dose of 12 x 10⁶ ± about 5% or a dose collected from 12 x 10⁶ ± about 5% cells, or a dose of 14 x 10⁶ ± about 5% or a dose collected from 12 x 10⁶ ± about 5% cells, or a dose of 16 x 10⁶ ± about 5% or a dose collected from 16 x 10⁶ ± about 5% cells, or a dose of 18 x 10⁶ ± about 5% or a dose collected from 18 x 10⁶ ± about 5% cells, or a dose of 20 x 10⁶ ± about 5% or a dose collected from 20 x 10⁶ ± about 5% cells. In some embodiments, between 100 x 10⁶ - 210 x 10⁶ ± 5%, or about 100 x 10⁶ ± about 5%, or a dose of 120 x 10⁶ ± about 5% or a dose collected from 120 x 10⁶ ± about 5% cells, or a dose of 140 x 10⁶ ± about 5% or a dose collected from 140 x 10⁶ ± about 5% cells, or a dose of 160 x 10⁶ ± about 5% or a dose collected from 160 x 10⁶ ± about 5% cells, or a dose of 180 x 10⁶ ± about 5% or a dose collected from 180 x 10⁶ ± about 5% cells, or a dose of 200 x 10⁶ ± about 5% or a dose collected from 200 x 10⁶ ± about 5% cells, or a dose of 1 x 10⁶ ± about 5% or a dose collected from 1 x 10⁶ ± about 5% cells, or a dose of 1 x 10⁷ ± about 5% or a dose collected from 1 x 10⁷ ± about 5% cells, or a dose of 1 x 10⁸ ± about 5% or a dose collected from 1 x 10⁸ ± about 5% cells, or a dose of 1 x 10⁹ ± about 5% or a dose collected from 1 x 10⁹ ± about 5% cells.

In some embodiments, between 1 x 10⁶ - 1 x 10⁹ 10%. In some embodiments, between 1 x 10⁶ - 1 x 10⁸ 10%. In some embodiments, between 1 x 10⁶ - 1 x 10⁷ 10%. In some embodiments, between 1 x 10⁷ - 1 x 10⁹ 10%. In some embodiments, between 1 x 10⁸ - 1 x 10⁹ 10%. In some embodiments, between 10 x 10⁶ - 500 x 10⁶ ± 10%. In some embodiments, between 10 x 10⁶ - 210 x 10⁶ ± 10%, or about 10 x 10⁶ ± about 10%, or a dose of 12 x 10⁶ ± about 10% or a dose collected from 12 x 10⁶ ± about 10% cells, or a dose of 14 x 10⁶ ± about 10% or a dose collected from 12 x 10⁶ ± about 10% cells, or a dose of 16 x 10⁶ ± about 10% or a dose collected from 16 x 10⁶ ± about 10% cells, or a dose of 18 x 10⁶ ± about 10% or a dose collected from 18 x 10⁶ ± about 10% cells, or a dose of 20 x 10⁶ ± about 10% or a dose collected from 20 x 10⁶ ± about 10% cells. In some embodiments, between 100 x 10⁶ - 210 x 10⁶ ± 10%, or about 100 x 10⁶ ± about 10%, or a dose of 120 x 10⁶ ± about 10% or a dose collected from 120 x 10⁶ ± about 10% cells, or a dose of 140 x 10⁶ ± about 10% or a dose collected from 140 x 10⁶ ± about 10% cells, or a dose of 160 x 10⁶ ± about 10% or a dose collected from 160 x 10⁶ ± about 10% cells, or a dose of 180 x 10⁶ ± about 10% or a dose collected from 180 x 10⁶ ± about 10% cells, or a dose of 200 x 10⁶ ± about 10% or a dose collected from 200 x 10⁶ ± about 10% cells, or a dose of 1 x 10⁶ ± about 10% or a dose collected from 1 x 10⁶ ± about 10% cells, or a dose of 1 x 10⁷ ± about 10% or a dose collected from 1 x 10⁷ ± about 10% cells, or a dose of 1 x 10⁸ ± about 10% or a dose collected from 1 x 10⁸ ± about 10% cells, or a dose of 1 x 10⁹ ± about 10% or a dose collected from 1 x 10⁹ ± about 10% cells.

In some embodiments, between 1 x 10⁶ - 1 x 10⁹ 15%. In some embodiments, between 1 x 10⁶ - 1 x 10⁸ 15%. In some embodiments, between 1 x 10⁶ - 1 x 10⁷ 15%. In some embodiments, between 1 x 10⁷ - 1 x 10⁹ 15%. In some embodiments, between 1 x 10⁸ - 1 x 10⁹ 15%. In some embodiments, between 10 x 10⁶ - 500 x 10⁶ ± 15%. In some embodiments, between 10 x 10⁶ - 210 x 10⁶ ± 15%, or about 10 x 10⁶ ± about 15%, or a dose of 12 x 10⁶ ± about 15% or a dose collected from 12 x 10⁶ ± about 15% cells, or a dose of 14 x 10⁶ ± about 15% or a dose collected from 12 x 10⁶ ± about 15% cells, or a dose of 16 x 10⁶ ± about 15% or a dose collected from 16 x 10⁶ ± about 15% cells, or a dose of 18 x 10⁶ ± about 15% or a dose collected from 18 x 10⁶ ± about 15% cells, or a dose of 20 x 10⁶ ± about 15% or a dose collected from 20 x 10⁶ ± about 15% cells. In some embodiments, between 100 x 10⁶ - 210 x 10⁶ ± 15%, or about 100 x 10⁶ ± about 15%, or a dose of 120 x 10⁶ ± about 15% or a dose collected from 120 x 10⁶ ± about 15% cells, or a dose of 140 x 10⁶ ± about 15% or a dose collected from 140 x 10⁶ ± about 15% cells, or a dose of 160 x 10⁶ ± about 15% or a dose collected from 160 x 10⁶ ± about 15% cells, or a dose of 180 x 10⁶ ± about 15% or a dose collected from 180 x 10⁶ ± about 15% cells, or a dose of 200 x 10⁶ ± about 15% or a dose collected from 200 x 10⁶ ± about 15% cells, or a dose of 1 x 10⁶ ± about 15% or a dose collected from 1 x 10⁶ ± about 15% cells, or a dose of 1 x 10⁷ ± about 15% or a dose collected from 1 x 10⁷ ± about 15% cells, or a dose of 1 x 10⁸ ± about 15% or a dose collected from 1 x 10⁸ ± about 15% cells, or a dose of 1 x 10⁹ ± about 15% or a dose collected from 1 x 10⁹ ± about 15% cells. More effective cells may be administered in even smaller numbers or collected from few cells, respectively. The precise determination of what would be considered an effective dose may be based on factors individual to each subject, including their size, age, sex, weight, condition of the particular subject, and site of application, for example the size of a synovial joint or area of bone being treated. Dosages can be readily ascertained by those skilled in the art from this disclosure and the knowledge in the art.

The skilled artisan can readily determine the number of cells, and optional additives, vehicles, and/or carrier in the compositions for use according to the present invention. Typically, any additives (in addition to the active cell(s) and/or agent(s)) are present in an amount of 0.001 to 50% (weight) solution in phosphate buffered saline, and the active ingredient is present in the order of micrograms to milligrams, such as about 0.0001 to about 5 wt %. In another embodiment about 0.0001 to about 1 wt %. In still another embodiment, about 0.0001 to about 0.05 wt% or about 0.001 to about 20 wt %. In a further embodiment, about 0.01 to about 10 wt %. In another embodiment, about 0.05 to about 5 wt %. Of course, for any composition to be administered to an animal or human, and for any particular type of administration, it is preferred to determine therefore: toxicity, such as by determining the lethal dose (LD) and LD50 in a suitable animal model e.g., rodent such as mouse; and, the dosage of the composition(s), concentration of components therein and timing of administering the composition(s), which elicit a suitable response. Such determinations do not require undue experimentation from the knowledge of the skilled artisan, this disclosure and the documents cited herein. The time for sequential administrations can be ascertained without undue experimentation.

### Methods of Therapeutic Uses

The present invention provides a composition comprising a mononuclear-enriched early apoptotic cell population comprising more than 40% Annexin V and less than 15% PI positive cells, wherein the cells are irradiated following the induction of apoptosis, for use in a method of treating osteoarthritis in a human subject by administering the composition directly into a joint of the subject.

A skilled artisan would appreciate that the term "joint disease or disorder" encompasses diseases in which the normal functioning or use of a joint is impaired. Impairment or loss of function may lead to discomfort or pain in or around the joint. Joint diseases and disorders may affect bone, joint capsule, cartilage, tendons, ligaments, tendon sheath, sac, synovial fluid, in or around the affected. Common diseases of the joints include osteoarthritis, rheumatoid arthritis, gout, lupus, tendonitis, bursitis, carpal tunnel syndrome, sprains, and other. In some embodiments, the joint disease or disorder comprises osteoarthritis.

In some embodiments, treating osteoarthritis comprises treating inflammatory osteoarthritis. In some embodiments, treating osteoarthritis comprises treating erosive osteoarthritis (EOA). In some embodiments, treating osteoarthritis comprises treating Gorham disease with inflammatory osteoarthritis. In some embodiments, osteoarthritis comprises inflammatory arthritis, erosive osteoarthritis, and vanishing bone. In some embodiments, osteoarthritis comprises any combination of inflammatory arthritis, erosive osteoarthritis, and vanishing bone. In some embodiments, osteoarthritis comprises inflammatory arthritis, or erosive osteoarthritis, or vanishing bone, or a combination thereof.

In some embodiments, treating of osteoarthritis comprises pain reduction, reduction of inflammation, reduction of swelling, inhibition of progressive degeneration of articular cartilage, reduction of progressive degeneration of articular cartilage, inhibition of erosion of bone tissue, or slowing of erosion of bone tissue, reduction in bone fractures, reduction of broken bones, inhibition of bone fractures, inhibition of broken bones, improving a quality of life, or any combination thereof in said subject suffering from the osteoarthritis. In some embodiments, treating osteoarthritis rebalances the immune response within a joint or a bone tissue, or in the adjacent tissue thereof, or any combination thereof.

In some embodiments, the treatment disclosed herein is a long-term treatment. In some embodiments, the effects of treatment last at least one-month post administration. In some embodiments, the effects of treatment last at least two months post administration. In some embodiments, the effects of treatment last at least 3, 4, 5, 6, 7, 8, 9, 10, or 11 months post administration. In some embodiments, the effects of treatment last between at least one-six months post administration. In some embodiments, the effects of treatment last between at least one-month and one-year post administration. In some embodiments, the effects of treatment last at least one-year post administration. In some embodiments, the effects of treatment last more than one-year post administration.

Osteoarthritis often affects synovial joints, such as at the knees, hips, shoulders, elbows, ankles, wrists, fingers, thumbs, neck, toes, thumb, hand, foot, or spine. Synovial joints consist of two bone ends covered by articular cartilage. Osteoarthritis may be caused by meniscal or ligament injury, pyogenic infection, ligamentous instability, joint fracture, obesity, or natural degenerative causes. Osteoarthritis often includes a progressive degeneration of articular cartilage at or in these joints. Joints affected by osteoarthritis may be painful, inflamed, swollen, and have a decreased range of movement.

Examples of the effects of osteoarthritis include the following: osteoarthritis in the hips can cause pain, stiffness, and severe disability. Patients may feel the pain in their hips, groin, inner thigh, buttocks, or knees. Osteoarthritis in the fingers may cause the fingers to become enlarged and gnarled. The disease may cause small, bony knobs to appear on the end joints of the fingers. Affected fingers may ache or be stiff and numb. More women than men suffer from osteoarthritis in the fingers, and they develop it especially after menopause. The base of the thumb joint may also be similarly affected by osteoarthritis. Osteoarthritis in the neck and spine may cause stiffness and pain in the neck or in the lower back. It may also cause weakness or numbness of the arms or legs. Osteoarthritis in the neck and spine is often debilitating and may result in the patient being bed-ridden.

One of the most common locations for osteoarthritis is in the knees. Osteoarthritis in the knee joint may cause the knee to be stiff, swollen, and painful-thus making it hard to walk, climb, and get in and out of chairs and bathtubs. If not treated, osteoarthritis in the knees can lead to permanent disability, and reduced quality of life.

In some embodiments, treating osteoarthritis in a subject in need, comprises treating osteoarthritis in a synovial joint. In some embodiments, treating osteoarthritis in a subject in need comprises treating osteoarthritis in a synovial joint selected from a knee joint, a hip joint, a shoulder joint, a joint between neck vertebrae, an elbow joint, an ankle joint, a wrist joint, a finger joint, a toe joint, a hand joint, a foot joint, or a thumb joint, or a combination thereof. In some embodiments, treating osteoarthritis in a subject in need comprises treating osteoarthritis in a knee joint. In some embodiments, treating osteoarthritis in a subject in need comprises treating osteoarthritis in a hip joint. In some embodiments, treating osteoarthritis in a subject in need comprises treating osteoarthritis in a shoulder joint. In some embodiments, treating osteoarthritis in a subject in need comprises treating osteoarthritis in a joint between neck vertebrae. In some embodiments, treating osteoarthritis in a subject in need comprises treating osteoarthritis in an elbow joint. In some embodiments, treating osteoarthritis in a subject in need comprises treating osteoarthritis in an ankle joint. In some embodiments, treating osteoarthritis in a subject in need comprises treating osteoarthritis in a wrist joint. In some embodiments, treating osteoarthritis in a subject in need comprises treating osteoarthritis in a finger joint. In some embodiments, treating osteoarthritis in a subject in need comprises treating osteoarthritis in a toe joint. In some embodiments, treating osteoarthritis in a subject in need comprises treating osteoarthritis in a hand joint. In some embodiments, treating osteoarthritis in a subject in need, comprises treating osteoarthritis in a foot joint. In some embodiments, treating osteoarthritis in a subject in need comprises treating osteoarthritis in a thumb joint. In some embodiments, treating osteoarthritis in a subject in need comprises treating osteoarthritis in a combination of synovial joints.

A skilled artisan would appreciate that the term "treating", and grammatical variations thereof, may encompass both therapeutic treatment and prophylactic or preventative measures, wherein the object is to prevent or lessen the targeted pathologic condition or disorder of osteoarthritis. In some embodiments, treating may include directly affecting or curing, suppressing, inhibiting, preventing, reducing the severity of, delaying the onset of, reducing symptoms associated with osteoarthritis. In some embodiments, "treating" may encompass delaying progression, speeding recovery, increasing efficacy of or decreasing resistance to alternative therapeutics, or a combination thereof. In some embodiments, "preventing" comprises delaying the onset of symptoms, preventing relapse to osteoarthritis, decreasing the number or frequency of relapse episodes, increasing latency between symptomatic episodes, or a combination thereof. In some embodiments, "suppressing" or "inhibiting", comprises reducing the severity of symptoms, reducing the severity of an acute episode, reducing the number of symptoms, reducing the incidence of disease-related symptoms, reducing the latency of symptoms, ameliorating symptoms, reducing secondary symptoms, reducing secondary infections, prolonging patient survival, or a combination thereof.

In some embodiments, treating osteoarthritis comprises pain reduction, reduction of inflammation, reduction of swelling, inhibition of progressive degeneration of articular cartilage, reduction of progressive degeneration of articular cartilage, or any combination thereof in and or around the joint being treated. Joint function can be measured by evaluating the parameters such as the presence of discomfort or pain during the range of motion and movement, and by evaluating the range of motion itself.

In some embodiments, treating osteoarthritis comprises pain reduction in and or around the treated joint. In some embodiments, treatment alleviates pain localized around the joint. Alleviating or reducing pain may in some embodiments, be associated with a reduction or alleviation of pain during movement of a joint. Alleviating or reducing pain may in some embodiments, be associated with a reduction or alleviation of pain in a joint under static conditions. In some embodiments, treating osteoarthritis comprises reduction of stiffness in and or around the joint being treated.

In some embodiments, reduction or alleviation of pain allows for increased range of movement of the joint. In some embodiments, directly administering early apoptotic cells increases movement in the treated joint. In some embodiments, the increased movement comprises increased range of movement or increased movement with reduced pain, or a combination thereof. In some embodiments, the increased movement comprises increased range of movement. In some embodiments, the increased movement is accompanied by reduced pain.

In some embodiments, treating osteoarthritis comprises reduction of inflammation in and or around the treated joint. In some embodiments, treatment leading to reduced inflammation may be the result of reduction in the secretion of proinflammatory cytokines and chemokines. For example, reduction of IL-1, IL-6, IL-8, IL-10, IL-1β, IL-2, IL-15, IL-22, MIP-1β, MCP-1, MDC, IP-10, fractalkine, IL-9, or TNFα, or any combination thereof. In some embodiments, treatment of osteoarthritis by direct administration of early apoptotic cells reduces pro- and anti-inflammatory cytokines and chemokines in the synovial fluid associated with the joint being treated. In some embodiments, the pro-inflammatory cytokines and chemokines reduced following treatment with early apoptotic cells comprises any proinflammatory cytokine or chemokine.

A skilled artisan would recognize that a measure of treatment effectiveness may be performed by measuring the level of cytokines and or chemokines in the synovial fluid associated with the joint being treated. Methods of measuring the presence and concentration of cytokines and or chemokines are well known in the art, and any known method could be used. In some embodiments, synovial fluid will be collected in order to measure different cytokines and or chemokines in the joint fluid. In some embodiments, synovial fluid will be collected in order to measure different metalloproteinases in the joint fluid. In some embodiments, synovial fluid will be collected in order to measure different pro-inflammatory cytokines in the joint fluid. In some embodiments, synovial fluid will be collected in order to measure different anti-inflammatory cytokines in the joint fluid. In some embodiments, synovial fluid will be collected in order to measure different pro- and anti-inflammatory cytokines in the joint fluid. In some embodiments, synovial fluid will be collected in order to measure any of IL-1, IL-6, IL-8, IL-10, IL-1β, IL-2, IL-15, IL-22, MIP-1β, MCP-1, MDC, IP-10, fractalkine, IL-9, or TNFα, or any combination thereof, in the joint fluid.

In some embodiments, synovial fluid is collected and assayed prior to treatment. In some embodiments, synovial fluid is collected and assayed prior to treatment and during treatment. In some embodiments, synovial fluid is collected and assayed prior to treatment, during treatment, and as a follow-up once treatment has been completed.

In some embodiments, synovial biopsies will be taken prior to treatment. In some embodiments, synovial biopsies will be taken prior to and during treatment. In some embodiments, synovial biopsies will be taken prior to and during treatment, and as a follow-up once treatment has been completed.

In some embodiments, direct administration of a composition comprising early apoptotic cells reduces the level of pro and anti-inflammatory cytokines or chemokines in the synovial fluid associated with the joint being treated. In some embodiments, the pro-inflammatory cytokines and chemokines reduced following treatment with early apoptotic cells comprises any of IL-1, IL-6, IL-8, IL-10, IL-1β, IL-2, IL-15, IL-22, MIP-1β, MCP-1, MDC, IP-10, fractalkine, IL-9, or TNFα, or any combination thereof. In some embodiments, direct administration of a composition comprising early apoptotic cells modifies the level of pro and anti-inflammatory cytokines or chemokines in the synovial fluid associated with the joint being treated. In some embodiments, the pro-inflammatory cytokines and chemokines having their concentration altered following treatment with early apoptotic cells comprises any of IL-1, IL-6, IL-8, IL-10, IL-1β, IL-2, IL-15, IL-22, MIP-1β, MCP-1, MDC, IP-10, fractalkine, IL-9, or TNFα, or any combination thereof.

In some embodiments, direct administration of a composition comprising early apoptotic cells reduces the level of IL-1 in the synovial fluid associated with the joint being treated. In some embodiments, direct administration of a composition comprising early apoptotic cells reduces the level of IL-6 in the synovial fluid associated with the joint being treated. In some embodiments, direct administration of a composition comprising early apoptotic cells reduces the level of IL-8 in the synovial fluid associated with the joint being treated. In some embodiments, direct administration of a composition comprising early apoptotic cells reduces the level of IL-10 in the synovial fluid associated with the joint being treated. In some embodiments, by direct administration of a composition comprising early apoptotic cells reduces the level of IL-1β in the synovial fluid associated with the joint being treated. In some embodiments, direct administration of a composition comprising early apoptotic cells reduces the level of IL-2 in the synovial fluid associated with the joint being treated. In some embodiments, direct administration of a composition comprising early apoptotic cells reduces the level of IL-15 in the synovial fluid associated with the joint being treated. In some embodiments, direct administration of a composition comprising early apoptotic cells reduces the level of IL-22 in the synovial fluid associated with the joint being treated. In some embodiments, direct administration of a composition comprising early apoptotic cells reduces the level of IL-9 in the synovial fluid associated with the joint being treated. In some embodiments, direct administration of a composition comprising early apoptotic cells reduces the level of MIP-1β in the synovial fluid associated with the joint being treated. In some embodiments, direct administration of a composition comprising early apoptotic cells reduces the level of MCP-1 in the synovial fluid associated with the joint being treated. In some embodiments, direct administration of a composition comprising early apoptotic cells reduces the level of MDC in the synovial fluid associated with the joint being treated. In some embodiments, direct administration of a composition comprising early apoptotic cells reduces the level of IP-10 in the synovial fluid associated with the joint being treated. In some embodiments, direct administration of a composition comprising early apoptotic cells reduces the level of fractalkine in the synovial fluid associated with the joint being treated. In some embodiments, direct administration of a composition comprising early apoptotic cells reduces the level of TNFα in the synovial fluid associated with the joint being treated.

In some embodiments, direct administration of a composition comprising early apoptotic cells modifies the level of IL-1 in the synovial fluid associated with the joint being treated. In some embodiments, direct administration of a composition comprising early apoptotic cells modifies the level of IL-6 in the synovial fluid associated with the joint being treated. In some embodiments, direct administration of a composition comprising early apoptotic cells modifies the level of IL-8 in the synovial fluid associated with the joint being treated. In some embodiments, direct administration of a composition comprising early apoptotic cells modifies the level of IL-10 in the synovial fluid associated with the joint being treated. In some embodiments, direct administration of a composition comprising early apoptotic cells modifies the level of IL-1β in the synovial fluid associated with the joint being treated. In some embodiments, direct administration of a composition comprising early apoptotic cells modifies the level of IL-2 in the synovial fluid associated with the joint being treated. In some embodiments, direct administration of a composition comprising early apoptotic cells modifies the level of IL-15 in the synovial fluid associated with the joint being treated. In some embodiments, direct administration of a composition comprising early apoptotic cells modifies the level of IL-22 in the synovial fluid associated with the joint being treated. In some embodiments, direct administration of a composition comprising early apoptotic cells modifies the level of MIP-1β in the synovial fluid associated with the joint being treated. In some embodiments, direct administration of a composition comprising early apoptotic cells modifies the level of MCP-1 in the synovial fluid associated with the joint being treated. In some embodiments, direct administration of a composition comprising early apoptotic cells modifies the level of MDC in the synovial fluid associated with the joint being treated. In some embodiments, direct administration of a composition comprising early apoptotic cells modifies the level of IP-10 in the synovial fluid associated with the joint being treated. In some embodiments, direct administration of a composition comprising early apoptotic cells modifies the level of fractalkine in the synovial fluid associated with the joint being treated. In some embodiments, direct administration of a composition comprising early apoptotic cells modifies the level of TNFα in the synovial fluid associated with the joint being treated.

In some embodiments, reduction of cytokines or chemokines is by about 10%-90%. In some embodiments, reduction of cytokines or chemokines is by about 20%-80%. In some embodiments, reduction of cytokines or chemokines is by about 30%-70%. In some embodiments, reduction of cytokines or chemokines is by about 40%-60%. In some embodiments, reduction of cytokines or chemokines is by about 80%-99%. In some embodiments, reduction of cytokines or chemokines is by about 10%. In some embodiments, reduction of cytokines or chemokines is by about 20%. In some embodiments, reduction of cytokines or chemokines is by about 30%. In some embodiments, reduction of cytokines or chemokines is by about 40%. In some embodiments, reduction of cytokines or chemokines is by about 50%. In some embodiments, reduction of cytokines or chemokines is by about 60%. In some embodiments, reduction of cytokines or chemokines is by about 70%. In some embodiments, reduction of cytokines or chemokines is by about 80%. In some embodiments, reduction of cytokines or chemokines is by about 90%. In some embodiments, reduction of cytokines or chemokines is by about 95%. In some embodiments, reduction of cytokines or chemokines is by about 99%.

A skilled artisan would appreciate that each cytokine or chemokine may be reduced by a different percentage, wherein the reduction of each cytokine or chemokine is by about 10%-90%. In some embodiments, reduction of cytokines or chemokines is by about 20%-80%. In some embodiments, reduction of each cytokine or chemokine is by about 30%-70%. In some embodiments, reduction of each cytokine or chemokine is by about 40%-60%. In some embodiments, reduction of each cytokine or chemokine is by about 80%-99%. In some embodiments, reduction of each cytokine or chemokine is by about 10%. In some embodiments, reduction of each cytokine or chemokine is by about 20%. In some embodiments, reduction of each cytokine or chemokine is by about 30%. In some embodiments, reduction of each cytokine or chemokine is by about 40%. In some embodiments, reduction of each cytokine or chemokine is by about 50%. In some embodiments, reduction of each cytokine or chemokine is by about 60%. In some embodiments, reduction of each cytokine or chemokine is by about 70%. In some embodiments, reduction of each cytokine or chemokine is by about 80%. In some embodiments, reduction of each cytokine or chemokine is by about 90%. In some embodiments, reduction of each cytokine or chemokine is by about 95%. In some embodiments, reduction of each cytokine or chemokine is by about 99%.

In some embodiments, treating osteoarthritis comprises reduction of swelling in and or around the joint being treated. In some embodiments, treating osteoarthritis comprises inhibition of progressive degeneration of articular cartilage in the joint being treated. In some embodiments, treating osteoarthritis comprises reduction of progressive degeneration of articular cartilage in the joint being treated.

A skilled artisan would appreciate that inhibition of progressive degeneration may be measured using ultrasound or MRI or X ray analysis, or a combination thereof, wherein the size of the cartilage is measured. Further, pain scales used in the art for the amount of pain a subject is feeling may be used to gauge the benefit of treatment and reduction of pain. In addition, fluid analysis may be performed analyzing different components of test samples comprising for example synovial fluid or joint fluid, and measuring cytokine levels, or chemokine levels, or levels of metalloproteases, or any combination thereof.

In some embodiments, said joint comprises a synovial joint. In some embodiments, a synovial joint comprises a comprises a knee joint, a hip joint, a shoulder joint, a joint between neck vertebrae, an elbow joint, an ankle joint, a wrist joint, a finger joint, a toe joint, or a thumb joint, a hand joint, a foot joint, or a combination thereof. In some embodiments, a subject in need suffers at a single joint. In some embodiments, a subject in need suffers at multiple joint.

In some embodiments, administration of a composition comprising an early apoptotic cell population comprises direct administration into a synovial joint. In some embodiments, administration of a composition comprising an early apoptotic cell population comprises direct administration into a knee joint. In some embodiments, administration of a composition comprising an early apoptotic cell population comprises direct administration into a hip joint. In some embodiments, administration of a composition comprising an early apoptotic cell population comprises direct administration into a shoulder joint. In some embodiments, administration of a composition comprising an early apoptotic cell population comprises direct administration into a joint between neck vertebrae. In some embodiments, administration of a composition comprising an early apoptotic cell population comprises direct administration into an elbow joint. In some embodiments, administration of a composition comprising an early apoptotic cell population comprises direct administration into an ankle joint. In some embodiments, administration of a composition comprising an early apoptotic cell population comprises direct administration into a wrist joint. In some embodiments, administration of a composition comprising an early apoptotic cell population comprises direct administration into a finger joint. In some embodiments, administration of a composition comprising an early apoptotic cell population comprises direct administration into a toe joint. In some embodiments, administration of a composition comprising an early apoptotic cell population comprises direct administration into a thumb joint. In some embodiments, administration of a composition comprising an early apoptotic cell population comprises direct administration into a hand joint. In some embodiments, administration of a composition comprising an early apoptotic cell population comprises direct administration into a foot joint. In some embodiments, administration of a composition comprising an early apoptotic cell population comprises direct administration into a combination of synovial joints selected from comprises a knee joint, a hip joint, a shoulder joint, a joint between neck vertebrae, an elbow joint, an ankle joint, a wrist joint, a finger joint, a toe joint, a hand joint, a foot joint, or a thumb joint, or a combination thereof

A skilled artisan would appreciate that the term "direct" encompasses administration of the early apoptotic cells or a composition thereof, respectively, within the joint being treated or immediately adjacent to the joint being treated. A direct treatment at a joint is in contrast to systemic administration for example intravenous injection or intraperitoneal infusion. In some embodiments, administration of a composition comprising early apoptotic cell population for treatment of osteoarthritis does not comprise a systemic administration.

In some embodiments, direct administration comprises injection of a composition of early apoptotic cells into a joint. In some embodiments, direct administration comprises injection a composition of early apoptotic cells into the tissue adjacent to a joint. In some embodiments, direct administration comprises infusion of a composition of early apoptotic cells into a joint. In some embodiments, direct administration comprises infusion a composition of early apoptotic cells into the tissue adjacent to a joint.

In some embodiments, direct administration comprises infiltration of a composition of early apoptotic cells into a cartilage tissue at a joint. In some embodiments, direct administration comprises infiltration of a composition of early apoptotic cells into the cartilage tissue adjacent to a joint.

In some embodiments, treatment of osteoarthritis comprises pain reduction, reduction of inflammation, reduction of swelling, inhibition of progressive degeneration of articular cartilage, reduction of progressive degeneration of articular cartilage, inhibition of erosion of bone tissue, or slowing of erosion of bone tissue, or any combination thereof. Affected individuals experience progressive destruction and resorption of bone. Multiple bones may become involved. Areas commonly affected include the ribs, spine, pelvis, skull, collarbone (clavicle), shoulder, and jaw. Pain and swelling in the affected area may occur. Bones affected are prone to reduced bone mass (osteopenia) and fracture. The severity can vary from one person to another and the disorder can potentially cause disfigurement and functional disability of affected areas.

In some embodiments, treatment alleviates pain localized around the bone. Alleviating or reducing pain may in some embodiments, be associated with a reduction or alleviation of pain during movement of the bone or in the area of the bone. Alleviating or reducing pain may in some embodiments, be associated with a reduction or alleviation of pain in a bone under static conditions. In some embodiments, treating osteoarthritis comprises reduction of stiffness in and or around the bone being treated.

In some embodiments, reduction or alleviation of pain allows for increased range of movement of the bone or area adjacent to the bone. In some embodiments, the increased movement comprises increased range of movement or increased movement with reduced pain, or a combination thereof. In some embodiments, the increased movement comprises increased range of movement. In some embodiments, the increased movement is accompanied by reduced pain.

In some embodiments, reduction of cytokines or chemokines is by about 10%-90%. In some embodiments, reduction of cytokines or chemokines is by about 20%-80%. In some embodiments, reduction of cytokines or chemokines is by about 30%-70%. In some embodiments, reduction of cytokines or chemokines is by about 40%-60%. In some embodiments, reduction of cytokines or chemokines is by about 80%-99%. In some embodiments, reduction of cytokines or chemokines is by about 10%. In some embodiments, reduction of cytokines or chemokines is by about 20%. In some embodiments, reduction of cytokines or chemokines is by about 30%. In some embodiments, reduction of cytokines or chemokines is by about 40%. In some embodiments, reduction of cytokines or chemokines is by about 50%. In some embodiments, reduction of cytokines or chemokines is by about 60%. In some embodiments, reduction of cytokines or chemokines is by about 70%. In some embodiments, reduction of cytokines or chemokines is by about 80%. In some embodiments, reduction of cytokines or chemokines is by about 90%. In some embodiments, reduction of cytokines or chemokines is by about 95%. In some embodiments, reduction of cytokines or chemokines is by about 99%.

A skilled artisan would appreciate that each cytokine or chemokine may be reduced by a different percentage, wherein the reduction of each cytokine or chemokine is by about 10%-90%. In some embodiments, reduction of cytokines or chemokines is by about 20%-80%. In some embodiments, reduction of each cytokine or chemokine is by about 30%-70%. In some embodiments, reduction of each cytokine or chemokine is by about 40%-60%. In some embodiments, reduction of each cytokine or chemokine is by about 80%-99%. In some embodiments, reduction of each cytokine or chemokine is by about 10%. In some embodiments, reduction of each cytokine or chemokine is by about 20%. In some embodiments, reduction of each cytokine or chemokine is by about 30%. In some embodiments, reduction of each cytokine or chemokine is by about 40%. In some embodiments, reduction of each cytokine or chemokine is by about 50%. In some embodiments, reduction of each cytokine or chemokine is by about 60%. In some embodiments, reduction of each cytokine or chemokine is by about 70%. In some embodiments, reduction of each cytokine or chemokine is by about 80%. In some embodiments, reduction of each cytokine or chemokine is by about 90%. In some embodiments, reduction of each cytokine or chemokine is by about 95%. In some embodiments, reduction of each cytokine or chemokine is by about 99%.

In some embodiments, administering a composition of early apoptotic cells reduces the concentration of at least one pro-inflammatory cytokine or chemokine in the synovial fluid present in the joint. In some embodiments, administering a composition of early apoptotic cells reduces the concentration of at least one pro-inflammatory cytokine or chemokine secreted from cells adjacent to an affected bone. In some embodiments, administering a composition of early apoptotic cells reduces the concentration of at least one anti-inflammatory cytokine or chemokine in the synovial fluid present in the joint. In some embodiments, administering a composition of early apoptotic cells reduces the concentration of at least one anti-inflammatory cytokine or chemokine secreted from cells adjacent to an affected bone. In some embodiments, administering a composition of early apoptotic cells reduces the concentration of at least one pro-inflammatory cytokine or chemokine and one anti-inflammatory cytokine or chemokine in the synovial fluid present in the joint. In some embodiments administering a composition of early apoptotic cells reduces the concentration of at least one pro-inflammatory cytokine or chemokine and one anti-inflammatory cytokine or chemokines secreted from cells adjacent to an affected bone.

In some embodiments, administration of a composition comprising an early apoptotic cell population comprises direct administration at the site of a shoulder bone, the skull, the pelvic girdle or a portion thereof, the jaw, a rib or ribs, the collar bone or a portion thereof, or the spine, or a combination thereof. In some embodiments, administration of a composition comprising an early apoptotic cell population comprises direct administration at the site of a shoulder bone. In some embodiments, administration of a composition comprising an early apoptotic cell population comprises direct administration at the site of the skull. In some embodiments, administration of a composition comprising an early apoptotic cell population comprises direct administration at the site of the pelvic girdle or a portion thereof. In some embodiments, administration of a composition comprising an early apoptotic cell population comprises direct administration at the site of the jaw or a portion thereof. In some embodiments, administration of a composition comprising an early apoptotic cell population comprises direct administration at the site of a rib or ribs. In some embodiments, administration of a composition comprising an early apoptotic cell population comprises direct administration at the site of the collar bone or a portion thereof. In some embodiments, administration of a composition comprising an early apoptotic cell population comprises direct administration at the site of or the spine or a portion thereof.

A skilled artisan would appreciate that the term "direct" encompasses administration of the early apoptotic cells or a composition thereof respectively, at or adjacent to the bone being treated. A direct treatment at a bone is in contrast to systemic administration for example intravenous injection or intraperitoneal infusion.

In some embodiments, direct administration comprises injection of a composition of early apoptotic cells at the site of an affected bone. In some embodiments, direct administration comprises injection a composition of early apoptotic cells adjacent to the site of an affected bone. In some embodiments, direct administration comprises infusion of a composition of early apoptotic cells at the site of an affected bone. In some embodiments, direct administration comprises infusion a composition of early apoptotic cells at the site adjacent to an affected bone.

In some embodiments, treating osteoarthritis comprises direct administration into the joint or at the site of the affecting bone, said administration comprising infusion or injection of said early apoptotic cell population.

Early apoptotic cell populations have been described in detail above including methods of preparing early apoptotic cells and compositions thereof, and therapeutically effective doses thereof. In some embodiments, any of the early apoptotic cell populations or compositions thereof described above, including for example ApoCells, Allocetra, Allocetra-OTS, Autocetra, or Autocetra-OTS may be utilized. In some embodiments, early apoptotic cells comprise allogeneic or autologous cells that have been irradiated.

In some embodiments, the early apoptotic cell population comprises an autologous early apoptotic cell population or an allogeneic early apoptotic cell population. The early apoptotic cells populations may in some embodiments be stable at early apoptotic stage for an extended time period of greater than 24 hours. In some embodiments, the early apoptotic cell population comprises an autologous early apoptotic cell population that is stable for greater than 24 hours; or an allogeneic early apoptotic cell population that is stable for greater than 24 hours.

In an embodiment, treating osteoarthritis with a composition comprising an early apoptotic cell population comprises use of single source or multiple source of mononuclear enriched cells. In some embodiments, the early apoptotic cells comprise a pooled population of early apoptotic cells. In some embodiments, the early apoptotic cells comprise a pooled population of early apoptotic cells, wherein the starting population of mononuclear enriched cells were obtained from a single source. In some embodiments, the early apoptotic cells comprise a pooled population of early apoptotic cells, wherein the starting population of mononuclear enriched cells were obtained from multiple sources. Cells may be pooled prior to or after induction of apoptosis. In some embodiments, the single source of cells comprises cells from an unmatched donor.

In some embodiments, the cells are irradiated in a way that will decrease proliferation and/or activation of residual viable cells within the apoptotic cell population. In some embodiments, the cells are irradiated in a way that reduces the percent of viable non-apoptotic cells in a population. In some embodiments, the percent of viable non-apoptotic cells in an inactivated early apoptotic cell population is reduced to less than 50% of the population. In some embodiments, the percent of viable non-apoptotic cells in an inactivated early apoptotic cell population is reduced to less than 40% of the population. In some embodiments, the percent of viable non-apoptotic cells in an inactivated early apoptotic cell population is reduced to less than 30% of the population. In some embodiments, the percent of viable non-apoptotic cells in an inactivated early apoptotic cell population is reduced to less than 20% of the population. In some embodiments, the percent of viable non-apoptotic cells in an inactivated early apoptotic cell population is reduced to less than 10% of the population. In some embodiments, the percent of viable non-apoptotic cells in an inactivated early apoptotic cell population is reduced to 0% of the population.

In another embodiment, the irradiated apoptotic cells preserve all their early apoptotic-, immune modulation-, stability-properties. In another embodiment, the irradiation step uses UV radiation. In another embodiment, the radiation step uses gamma radiation. In another embodiment, the apoptotic cells comprise a decreased percent of living non-apoptotic cells, comprise a preparation having a suppressed cellular activation of any living non-apoptotic cells present within the apoptotic cell preparation, or comprise a preparation having reduced proliferation of any living non-apoptotic cells present within the apoptotic cell preparation, or any combination thereof.

In some embodiments, a cell population comprising a reduced or non-existent fraction of living non-apoptotic cells may provide a mononuclear early apoptotic cell population that does not have any living / viable cells.

In some embodiments, use of irradiated ApoCells as shown here in the Examples (See Example 3 for use of single source autologous irradiated early apoptotic cell population) result from the apoptotic cells and not from a viable proliferating population of cells with cellular activity, present within the apoptotic cell population.

In some embodiments, a pooled mononuclear apoptotic cell preparation comprises mononuclear cells in an early apoptotic state, wherein said pooled mononuclear apoptotic cells comprise a decreased percent of living non-apoptotic cells, a preparation having a suppressed cellular activation of any living non-apoptotic cells, or a preparation having reduced proliferation of any living non-apoptotic cells, or any combination thereof. In another embodiment, disclosed herein is a pooled mononuclear apoptotic cell preparation that in some embodiments, originates from the white blood cell fraction (WBC) obtained from donated blood.

In another embodiment, said irradiation comprises gamma irradiation or UV irradiation. In yet another embodiment, the irradiated preparation has a reduced number of non-apoptotic cells compared with a non-irradiated apoptotic cell preparation. In another embodiment, the irradiated preparation has a reduced number of proliferating cells compared with a non-irradiated apoptotic cell preparation. In another embodiment, the irradiated preparation has a reduced number of potentially immunologically active cells compared with a non-irradiated apoptotic cell population.

In some embodiments, early apoptotic cells are prepared from pooled blood collected from a single autologous donor or from multiple donors, prepared and possibly stored for later use. This combined pool of blood may then be processed to produce a pooled mononuclear apoptotic cell preparation. In another embodiment, a pooled mononuclear apoptotic cell preparation ensures that a readily available supply of mononuclear apoptotic cells is available for use in treating osteoarthritis.

In some embodiments, treating osteoarthritis comprises direct administration of a composition comprising a pooled early apoptotic cell population that comprises apoptotic cells prepared from single donor. In some embodiments, treating osteoarthritis comprises direct administration of a composition comprising a pooled early apoptotic cell population that comprises apoptotic cells prepared from multiple donor mononuclear cells. In some embodiments, treating osteoarthritis comprises direct administration of a composition comprising a pooled early apoptotic cell population that comprises apoptotic cells prepared from a single source donor or multiple donor mononuclear cells.

In some embodiments, the subject is a mammalian subject. In some embodiments, the subject is a human subject. In some embodiments, the human subject is a child. In some embodiments, the human subject is an adult.

Dosages of early apoptotic cells have been described in detail above. In some embodiments, the direct administering of a composition of early apoptotic cells comprises a single administration of the early apoptotic cell population cells. In some embodiments, the direct administering of a composition of early apoptotic cells comprises multiple administrations of said apoptotic cell population. In some embodiments, the single direct administration is into an affected joint. In some embodiments, the single direct administration is into tissue as close as possible to an affected bone. In some embodiments, the single direct administration is adjacent to an affected joint. In some embodiments, the single direct administration is adjacent to an affected bone. In some embodiments, the multiple direct administrations are into an affected joint. In some embodiments, the multiple direct administrations are into tissue as close as possible to an affected bone. In some embodiments, the multiple direct administrations are adjacent to an affected joint. In some embodiments, the multiple direct administrations are adjacent to an affected bone. In some embodiments, direct multiple administrations comprise into a joint and adjacent to the joint. In some embodiments, direct multiple administrations comprise into tissue as close as possible to an affected bone and adjacent to the affected bone.

Dosage regimes have been described in detail above. In an embodiment, a dosage regime as described above with the early apoptotic cells may be used.

In some embodiments, the composition of early apoptotic cells is administered daily or weekly. In some embodiments, the composition of early apoptotic cells comprises a dose of apoptotic cells that is administered weekly. In some embodiments, a dose of apoptotic cells is administered semi-weekly (twice a week). In some embodiments, a dose of apoptotic cells is administered bi-weekly (every two weeks). In some embodiments, a dose of apoptotic cells is administered monthly. In some embodiments, a dose of apoptotic cells is administered in a non-regular regime, for example daily for a given time period followed by semi-weekly, or weekly, or bi-weekly, or monthly administration, or a combination thereof.

In some embodiments, a dose of about 1 × 10⁶ - 1 × 10⁸ apoptotic cells is administered. In some embodiments, a dose of about 1 × 10⁶ - 1 × 10⁷ apoptotic cells is administered. In some embodiments, a dose of about 1 × 10⁷ - 1 × 10⁹ apoptotic cells is administered. In some embodiments, a dose of about 1 × 10⁷- 1 × 10⁸ apoptotic cells is administered. In some embodiments, a dose of about 1 × 10⁸ - 1 × 10⁹ apoptotic cells is administered. In some embodiments, a dose of about 1 × 10⁶ apoptotic cells is administered. In some embodiments, a dose of about 1 × 10⁷ apoptotic cells is administered. In some embodiments, a dose of about 1 × 10⁸ apoptotic cells is administered. In some embodiments, a dose of about 1 × 10⁹ apoptotic cells is administered.

In some embodiments, a dose of about 140 × 10⁶ - 210 × 10⁶ apoptotic cells is administered. In some embodiments, a dose of about 100 × 10⁶ - 140 × 10⁶ apoptotic cells is administered. In some embodiments, a dose of about 10-100 × 10⁶ apoptotic cells is administered. In some embodiments, a dose of about 20 x 10⁶ apoptotic cells is administered. In some embodiments, a dose of about 30 × 10⁶apoptotic cells is administered. In some embodiments, a dose of about 40 × 10⁶ apoptotic cells is administered. In some embodiments, a dose of about 50 × 10⁶ apoptotic cells is administered. In some embodiments, 60 × 10⁶ apoptotic cells is administered. In some embodiments, a dose of about 60 × 10⁶ apoptotic cells is administered. In some embodiments, a dose of about 70 × 10⁶ apoptotic cells is administered. In some embodiments, a dose of about 80 × 10⁶ apoptotic cells is administered. In some embodiments, a dose of about 90 × 10⁶ apoptotic cells is administered. In some embodiments, a dose of about 1-15 × 10⁷ apoptotic cells is administered. In some embodiments, a dose of about 10 × 10⁷ apoptotic cells is administered. In some embodiments, a dose of about 11 × 10⁷ apoptotic cells is administered. In some embodiments, a dose of about 12 × 10⁷ apoptotic cells is administered. In some embodiments, a dose of about 13 × 10⁷ apoptotic cells is administered. In some embodiments, a dose of about 14 × 10⁷ apoptotic cells is administered. In some embodiments, a dose of about 15 × 10⁷ apoptotic cells is administered. In some embodiments, a dose of 1×10⁶ apoptotic cells is administered. In some embodiments, a dose of 10×10⁶ apoptotic cells is administered. In another embodiment, a dose of 10×10⁷ apoptotic cells is administered. In another embodiment, a dose of 10x10⁸ apoptotic cells is administered. In another embodiment, a dose of 10x10⁹ apoptotic cells is administered. In another embodiment, a dose of 10x10¹⁰ apoptotic cells is administered. In another embodiment, a dose of 10x10¹¹ apoptotic cells is administered. In another embodiment, a dose of 10x10¹² apoptotic cells is administered. In another embodiment, a dose of 10x10⁵ apoptotic cells is administered. In another embodiment, a dose of 10x10⁴ apoptotic cells is administered. In another embodiment, a dose of 10x10³ apoptotic cells is administered. In another embodiment, a dose of 10x10² apoptotic cells is administered.

In some embodiments, a high dose of apoptotic cells is administered. In some embodiments, a lower dose of apoptotic cells, dependent on the size of the joint is administered. In some embodiments, a dose of 10x10⁶ apoptotic cells is administered. In some embodiments, a dose of 15x10⁶ apoptotic cells is administered. In some embodiments, a dose of 20x10⁶ apoptotic cells is administered. In some embodiments, a dose of 25x10⁶ apoptotic cells is administered. In some embodiments, a dose of 30x10⁶apoptotic cells is administered. In some embodiments, a dose of 35x10⁶ apoptotic cells is administered. In some embodiments, a dose of 50x10⁶ apoptotic cells is administered. In some embodiments, a dose of 100x10⁶ apoptotic cells is administered. In some embodiments, a dose of 150x10⁶ apoptotic cells is administered. In some embodiments, a dose of 200x10⁶ apoptotic cells is administered. In another embodiment, a dose of 210x10⁶ apoptotic cells is administered. In another embodiment, a dose of 70x10⁶ apoptotic cells is administered. In another embodiment, a dose of 140x10⁶apoptotic cells is administered. In another embodiment, a dose of 35-210x10⁶ apoptotic cells is administered.

In some embodiments, the number of early apoptotic cells is ± 5%, 10%, 15%, or 20% of the number of cells. In some embodiments, the number of apoptotic cells is ± 5%, 10%, 15%, or 20% of the number of cells. For example, the dose of each administration comprises between about 50 x10⁶±5% to 200 x 10⁶ ±5% early apoptotic cells/kg subject, or 50 x 106 ±10% to 200 x 106 ±10% early apoptotic cells/kg subject, or 50 x 10⁶ ±15% to 200 x 10⁶ ±15% early apoptotic cells/kg subject, or 50 x 10⁶ ±20% to 200 x 10⁶ ±20% early apoptotic cells/kg subject.

### EXAMPLES

### EXAMPLE 1: Apoptotic Cell Production

Objective: To produce early-apoptotic cells, including irradiated early apoptotic cells.

Methods: Methods of making populations of early-apoptotic cells have been well documented in International Publication No. WO 2014/087408 and United States Application Publication No. US2015/0275175-A1, see for example, the Methods section preceding the Examples at "Early apoptotic cell population Preparation" and "Generation of apoptotic cells" (paragraphs [0223] through [0288]), and Examples 11, 12, 13, and 14).

The flow chart presented in Figure 1 provides an overview of one embodiment of the steps used during the process of producing a population of early apoptotic cells, wherein anticoagulants were included in the thawing and induction of apoptosis steps. As is described in detailed in Example 14 of International Publication No. WO 2014/087408 and United States Application Publication No. US-2015-0275175-A1, early apoptotic cell populations were prepared wherein anti-coagulants were added at the time of freezing, or at the time of incubation, or at the time of freezing and at the time of incubation. The anticoagulant used was acid-citrate dextrose, NIH Formula A (ACD formula A) was supplemented with 10 U/ml heparin to a final concentration of 5% ACD of the total volume and 0.5 U/ml heparin.

The population of cells used as a starting population may vary in certain embodiments, yet methods producing the early apoptotic cell described herein result in the final products having the same qualities and characteristics. In some embodiments, cells used to make early apoptotic cells may be autologous, allogeneic from a donor, or allogeneic from a blood bank.

Briefly: The cells were collected and then frozen with addition of 5% anticoagulant citrate dextrose formula A and 10U/ml heparin (ACDhep) to the freezing media. Thawing, incubation in an apoptosis induction media containing 5% ACDhep, and final product preparation were performed in a closed system.

Apoptosis and viability analysis, potency assay, and cell population characterization were performed in each experiment. In order to establish consistence in production of the early apoptotic cell product, the final product (FP) of initial batches of apoptotic cells were stored at 2-8°C and examined at t0, t24h, t48h and t72h. At each point apoptosis analysis, short potency assay (Applicants CD14+ frozen cells), trypan blue measurement and cell population characterization were performed. The FP was tested for cell count to assess average cell loss during storage and apoptosis and viability analysis.

The methods sections cited above and Example 11 of International Publication No. WO 2014/087408 and United States Application Publication No. US-2015-0275175-A1 provide details of preparing other embodiment of apoptotic cell populations in the absence of anti-coagulants.

In certain instances, the early apoptotic cells were irradiated after they were prepared (after induction of apoptosis), in other words following the last step shown in Figure 1. In other embodiments, irradiation could occur at an earlier step of the procedure to produce irradiated early apoptotic cells.

Methods of preparing irradiated apoptotic cells: Similar methods were used to prepare an inactivated apoptotic cell population, wherein a mononuclear early apoptotic cell population, single source or from multiple sources, comprises a decreased percent of non-quiescent non-apoptotic cells, or a population of cells having a suppressed cellular activation of any living non-apoptotic cells, or a population of cells having a reduced proliferation of any living non-apoptotic cells, or any combination thereof.

Briefly, an enriched mononuclear cell fraction was collected via leukapheresis procedure from healthy, eligible donors. Following apheresis completion, cells were washed and resuspended with freezing media comprising 5% Anticoagulant Citrate Dextrose Solution-Formula A (ACD-A) and 0.5U\ml heparin. Cell were then gradually frozen and transferred to liquid nitrogen for long term storage. In some embodiments, multiple fractions from different donors or the same donor were pooled.

For preparation of irradiated ApoCells (early apoptotic cells), cryopreserved cells were thawed, washed and resuspended with apoptosis induction media comprising 5% ACD-A, 0.5U\ml heparin sodium and 50µg/ml methylprednisolone. Cells were then incubated for 6 hours at 37°C in 5% CO₂. At the end of incubation, cells were collected, washed and resuspended in Hartmann's solution using a cell processing system (Fresenius Kabi, Germany). In some embodiments, collected apoptotic cell fractions were pooled to create a pooled, apoptotic cell fraction. Following manufacturing completion (induction of apoptosis), ApoCells were irradiated at 4000 cGy using g-camera at the radiotherapy unit, Hadassah Ein Kerem. Apoptosis and viability of ApoCell determined using AnnexinV and PI (MBL, MA, USA) staining (≥ 40% and ≤ 15%, respectively) via Flow cytometer. Results analyzed using FCS express software. In some embodiments, following irradiation, single source irradiated, apoptotic cells were pooled, wherein the source was the same or different donors. In other embodiments, multiple single source or multiple different source apoptotic cells were pooled prior to irradiation.

This irradiated ApoCell population is considered to include early apoptotic cells, wherein any viable cells present have suppressed cellular activity and reduced or no proliferation capabilities. In certain cases, the ApoCell population has no viable non-apoptotic cells.

### Results:

The stability of the FP produced with inclusion of anticoagulant at freezing and incubation (apoptotic induction) and then stored at 2-8°C are shown below in Table 1.

**Table 1: Cell count*- performed using a MICROS 60 hematology analyzer.**

| FP Time point | Cell concentration (x10⁶cells\ml) | % of cell loss |
|---|---|---|
| t0 | 20.8 | NA |
| t24h | 20.0 | -3.85 |
| t48h | 20.0 | -3.85 |
| t72h | 19.7 | -5.3 |

| | | |
|---|---|---|
| * Results Representative of 6 (six) experiments. | | |

When manufacturing the cells without including an anticoagulant in the induction medium, cells were stable for 24 hours and less stable thereafter. Use of anticoagulants unexpectedly extended the stability of the apoptotic cell population for at least 72 hours, as shown in Table 1.

**Table 2: Trypan blue measurement**

| FP Time point | trypan blue positive cells (%) |
|---|---|
| t0 | 3.0 |
| t24h | 5.9 |
| t48h | 5.2 |
| t72h | 6.5 |

The results of Table 2 show viability of the FP remained high for at least 72 hours.

**Table 3: Apoptosis analysis- (AnPI staining) performed using Flow Cytometry**

| FP Time point | 1.5mM Ca | | |
|---|---|---|---|
| | An-PI- (%) | An+PI- (%) | An+PI+ (%) |
| t0 | 44.3 | 50.9 | 4.8 |
| t24h | 39.0 | 55.9 | 5.1 |
| t48h | 34.8 | 60.1 | 5.1 |
| t72h | 33.4 | 60.5 | 6.1 |

The results of Table 3 show that the percent apoptotic cells versus necrotic cells was maintained over at extended time period of at least 72 hours post preparation of the cells, as was the percentage of early apoptotic cells.

The data in Table 3 confirms that the majority of cells in the population produced are in early apoptosis, wherein the percent of cells in the population in early apoptosis (An+PI-) was greater than 50% and in some instances greater than 60%. The cell population produced comprises a minimal percent of cells in late apoptosis or dead cells (less than or equal to 6%). See also Table 5 below.

Inclusion of anticoagulants both at the time of freezing and during induction of apoptosis resulted in the most consistently high yield of stable early-apoptotic cells (average yield of early apoptotic cells 61.3±2.6% % (An+PI-) versus 48.4±5.0%, wherein 100% yield is based on the number of cells at freezing). This high yield was maintained even after 72 hours storage at 2-8°C.

Next a comparison was made between the inclusion of the anticoagulant at freezing or thawing or both, wherein percent (%) recovery was measured as well as stability. Anticoagulant was included in the apoptotic incubation mix for all populations. Table 4 presents the results of these studies.

**Table 4: Yield and stability comparison of final products (FP) manufactured from cells collected, with ("+") or without ("-") addition of anticoagulant during freezing ("F") and thawing ("Tha")**

| Donor ID | # of Collected Cells (×10⁹, 100%) | % Cell Recovery in Final Product of Collected Cells | | | | | | | |
|---|---|---|---|---|---|---|---|---|---|
| | | FP t0 | | | | FP t24h* | | | |
| | | | **F-/Tha+** | **F+/Tha+** | **F+/Tha-** | **F-/Tha-** | **F-/Tha+** | **F+/Tha+** | **F+/Tha-** |
| **1** | 13.3 | 52.1 | 53.4 | 62.5 | 62 | 52.1 | 48.9 | 62.5 | 62 |
| **2** | 13.6 | 50.5 | 36.7 | F-/Tha- | 63.5 | 47.6 | 36.7 | 53.1 | 59.7 |
| **3** | 15.0 | 42.7 | 42 | 53.6 | 58.4 | 42.7 | 41.7 | 53.6 | 57.8 |
| **Avg** | 14.0 | 48.4±5.0 | 44.0±8.5 | 56.5±5.2 | 61.3±2.6 | 47.5±4.7 | 42.4±6.1 | 56.4±5.3 | 59.8±2.1 |

Additional population analysis comparisons of early apoptotic cell populations (batches of cells) prepared with and without anti-coagulant added, show the consistency of these results.

**Table 5: Cell population analysis comparison between batches prepared with and without anticoagulant.**

| Test | Specification | At | | ApoCell | | ApoCell | |
|---|---|---|---|---|---|---|---|
| | | Thawing | | Time 0 h | | Time 24 h | |
| | | | | | | Storage | |
| | | w\o ACDhep | +ACDhep | w\o ACDhep | +ACDhep | w\o ACDhep | +ACDhep |
| Change in Total Cell Count | >35.0% | 85.5 (79.5-92.5) | 82.8 (67.7-96.4) | 49.9 (46.6-52.3) | 66.7 (62.5-71.2) | 49.0 46.6-50.3) | 66.7 (62.5-71.2) |
| Percent change (min-max) | | | | | | | |
| Changes in ApoCell | 90.0±10.0% | | | 100 | 100 | 98.2 (96.2-100) | 100 |
| Percent change | | | | | | | |
| Range (min-max) | | | | | | | |
| Cell viability PI exclusion | > 85.0% | 98.0 (97.4-98.4) | 96.0 (91.9-98.1) | 98.5 (97.9-99.2) | 94.6 (93.5-95.5) | 97.7 (96.4-98.6) | 94.5 (93.4-95.1) |
| Percent viable | | | | | | | |
| Range (min-max) | | | | | | | |
| Identity/ Purity | CD3 (T cells): | 75.7 (71.6-81.4) | 66.5 (60.1-70.1) | 73.3 (70.3-78.3) | 62.8 (61.1-65.3) | 71.6 (61.5-79.1) | 64.2 (61.6-68.1) |
| | 71.9 (50.0-85.0) | | | | | | |
| Analysis of cell phenotype | ApoCell CD3: | | | | | | |
| Average (%) (maximal calculated range) | 71.6 (50.0-85.0) | | | | | | |
| | CD19 (B cells): | 7.5 (4.0-11.1) | 9.8 (8.6-12.0) | 9.0 (7.6-10.2) | 9.9 (9.3-10.2) | 9.5 (8.6-10.3) | 9.7 (9.2-10.4) |
| | 9.3 (3.0-15.0) | | | | | | |
| | ApoCell CD19: | | | | | | |
| | 9.5 (4-15) | | | | | | |
| | CD14 (monocytes): | 9.8 (6.4-13.0) | 14.0 (8.8-22.1) | 11.6 (10.2-13.3) | 15.4 (8.2-19.3) | 9.3 (4.8-17.2) | 16.1 (9.0-20.4) |
| | 10.1 (2.5-22.0) | | | | | | |
| | ApoCell CD14: | | | | | | |
| | 10.6 (2.5-22.0) | | | | | | |
| | CD15 ^{high} (granulocytes): | 0.2 (0-0.3) | 0.46 (0.18-0.69) | 0.2 (0.1-0.4) | 0.083 (0.08-0.09) | 0.1 (0.1-0.2) | 0.09 (0.07-0.1) |
| | 0.4 (0-6.0) | | | | | | |
| | ApoCell CD15^{high}: | | | | | | |
| | 0.2 (0-2.0) | | | | | | |
| | CD 56 (NK): | 7.4 (2.4-11.0) | 10.1 (6.6-14.2) | 4.7 (2.7-8.0) | 11.2 (7.2-14.2) | 4.9 (2.2-9.2) | 10.0 (6.4-13.0) |
| | 7.2 (1.5-22.0) | | | | | | |
| | ApoCell CD56: | | | | | | |
| | 5.2 (1.5-15.0) | | | | | | |

Percentage of final product cells (yield) in the presence or absence of anticoagulants. Similar to the results presented above at Table 1, the data presented in Table 4 demonstrates that early apoptotic cells manufactured from cells frozen in the presence of anticoagulant had a beneficial effect on average yield of fresh final product (FP t0) as compared to cells frozen without anticoagulant. The beneficial effect was seen when anticoagulant was used while freezing only (61.3±2.6% versus 48.4±5.0%), or both freezing and thawing (56.5±5.2% versus 48.4±5.0%). The beneficial effect was less significant when anticoagulant was used upon thawing only (44.0±8.5% versus 48.4±5.0%). These were non-high triglyceride samples.

Effect of anticoagulants on aggregation. No cell aggregations were seen in these 3 non-high triglyceride samples, or in 21 additional samples (data not shown). However, in 41 other non-high triglyceride samples manufactured without anticoagulants (data not shown), mild aggregates were seen in 10 (24.4%) and severe aggregates in 5 (12.2%); thus, anticoagulants avoid completely cell aggregates.

Effect of anticoagulants on stability. Fresh FPs manufactured with- or without anticoagulants were stored at 2-8°C for 24 hours to determine whether addition of ACDhep to the manufacturing procedure impairs the stability of the FP. Cells were sampled following 24 hours of storage and yield was calculated. Similar to the results shown in Table 1 for extended time periods (up to 72 hours), Table 4 shows that the beneficial effect was kept and observed when anticoagulant was used while freezing only (59.8±2.1% versus 47.5±4.7%), or both freezing and thawing (56.4±5.3% versus 47.5±4.7%). The beneficial effect was less significant when anticoagulant was added only upon thawing (42.4±6.1% versus 47.5±4.7%). These were all non-high triglyceride samples. These results show minimal cell loss following 24 hours of FP storage in all treatments with significant advantage to cells treated with anticoagulant during both freezing and thawing. Average loss of cells treated with anticoagulant during freezing only was 2.3±3.2% compared to 1.9±3.3% without anticoagulants, upon thawing only was 3.0±4.7 compared to 1.9±3.3% without anticoagulants, and 0.2±0.4% compared to 1.9±3.3% without anticoagulants when cells were both frozen and thawed with ACDhep. In summary, the beneficial effect of anticoagulants on yield was kept for at least 24 hours.

The characteristics of a representative cell population of the FP are shown below in Table 6.

**Table 6: Characterization of the cell population of fresh (t0) FP manufactured from cells collected with ("+") or without ("-") addition of anticoagulant during freezing ("F") and thawing ("Tha") procedures***

| | **FP t0** | | | | | | | | | |
|---|---|---|---|---|---|---|---|---|---|---|
| **DonorID *1-3*** | **F-/Tha-** | | | | | **F-/Tha+** | | | | |
| | CD3+ (%) | CD19+ (%) | CD56+ (%) | CD14+ (%) | CD15+ (%) | CD3+ (%) | CD19+ (%) | CD56+ (%) | CD14+ (%) | CD15+ (%) |
| | 62.2± 6.1 | 5.6± o.7 | 9.8± 0.9 | 13.5± 1.1 | 0± 0 | 61±6.1 | 8.6± 0.4 | 8.6± 0.9 | 14.1± 1.1 | 0± 0 |
| | **F+/Tha+** | | | | | **F+/Tha-** | | | | |
| | CD3+ (%) | CD19+ (%) | CD56+ (%) | CD14+ (%) | CD15+ (%) | CD3+ (%) | CD19+ (%) | CD56+ (%) | CD14+ (%) | CD15+ (%) |
| | 63.9± 5.8 | 7.4± 0.6 | 9.4± 0.8 | 13.3± 1.9 | 0± 0 | 61.9± 6.0 | 11.5± 1.1 | 10.1± 1.0 | 14.3± 1.3 | 0± 0 |

*Induction of apoptosis was performed using a medium containing anticoagulant for all batches.

The results of Table 6 show the cell characteristics of the final products (FP) manufactured with or without anticoagulant at freezing and thawing. Batches were sampled, stained for mononuclear markers, and analyzed via flow cytometry to determine the cell distribution in each sample and to examine whether the addition of anticoagulant affected the cell population. As presented in Table 5, there were no significant differences detected in cell populations manufactured with or without anticoagulants at freezing or thawing. The average T cell population (CD3+ cells) in fresh FP was 62.3±1.2% between treatments compared to 62.9±1.1% before freezing; the average B cell population (CD19+ cells) was 8.3±2.5% between treatments compared to 3.1±0.8% before freezing; the average natural killer cell population (CD56+ cells) was 9.5±0.7% between treatments compared to 12.9±0.5% before freezing; the average monocyte cell population (CD14+ cells) was 13.8±0.5% between treatments compared to 17.5±0.3% before freezing; and the average granulocyte population (CD15+ cells) was 0.0% in the fresh FP compared to 0.35±0.2% at freezing.

The potency of the early apoptotic population was also examined.

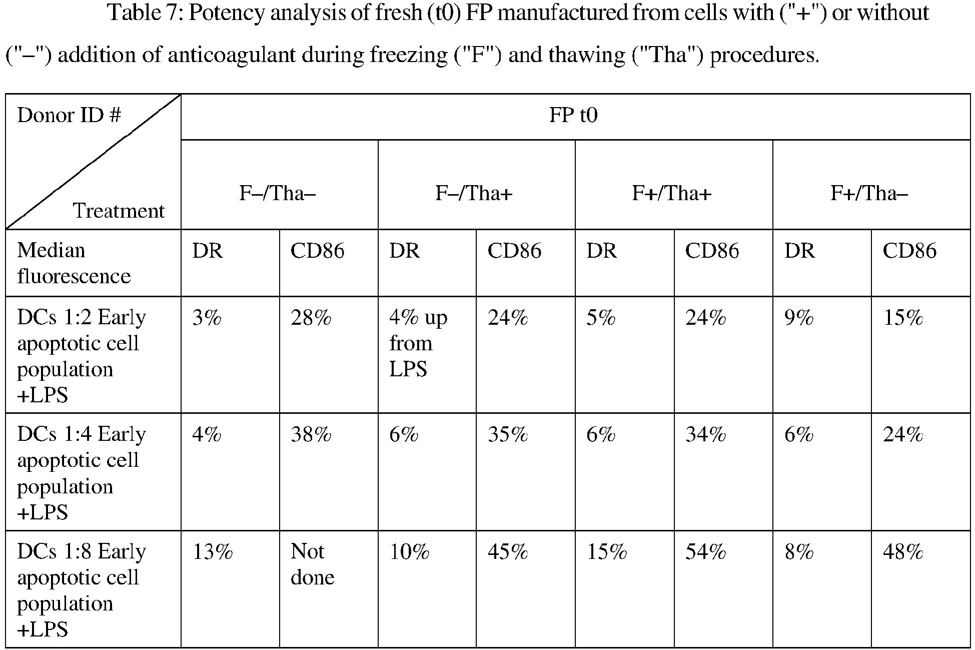

The results presented in Table 7 are from a potency assay performed to determine the ability of each final product to enhance a tolerogenic state in immature dendritic cells (iDCs) following stimulation with (LPS). The tolerogenic effect was determined by assessing downregulation of co-stimulatory molecule HLA-DR and CD86 expression on iDCs following interaction with the early apoptotic cell populations and different treatments leading to LPS upregulation. The analysis was performed on DCsign+ cells. Results represent the percent delay in maturation following interaction with early apoptotic cell population and following addition of LPS versus LPS-induced maturation. The experiment tested the potency of fresh FP (t0) manufactured with- or without anticoagulant. Results presented in Table 7 show that apoptotic cells manufactured with or without anticoagulant enhance the tolerance effect of both co-stimulatory markers in a dose-dependent manner.

The early apoptotic cells produced herein were from non-high triglyceride samples. This consistent high yield of stable early apoptotic cells was produced even in the cases when the donor plasma is high in triglycerides (See for example, Examples 12 and 13 of International Publication No. WO 2014/087408 and United States Application Publication No. US-2015-0275175-A1). Note that anti-coagulants were not added to the PBS media used for formulation of the final early apoptotic cell dose for infusion.

### Summary:

The objective of this study was to produce a stable, high yield early apoptotic cell population. The rational for use of anticoagulants was that aggregates were seen first in patients with high-triglycerides, but later in a significant portion of other patients. A concern here was the disclosure in United States Patent No. US 6,489,311 that the use of anticoagulants prevented cell apoptosis.

In short, with minimal impact on the composition, viability, stability, and the apoptotic nature of the cells, there was a significant improvement of at least 10-20% in the number of collected cells in the final product (Yield) when anticoagulant was added. In this study an up to 13% increase in yield was shown, which represents 26.8% augmentation in yield in controlled conditions but in real GMP conditions it went up to 33% and more augmentations in cell number then can be produced in a single collection. This effect is crucial, since it may avoid the need for a second apheresis from a donor.

This effect was surprising because the anticipated impact was expected to be dissolution of mild aggregates. It had been hypothesize that thawing cells with anticoagulant reduced the amount of aggregates. When formed, these aggregates eventually lead to massive cell loss. Cells collected and frozen without anticoagulant demonstrated aggregate formation at thawing, immediately after wash. Furthermore, a high level of aggregates was also detected in cells that were frozen without anticoagulant and resuspended with media containing anticoagulant. No aggregates were seen in cells that were both frozen and resuspended with media containing anticoagulant. Taken together, it was conclude that the addition of anticoagulants during freezing and apoptosis induction is of high importance, and did not appear to negatively impact the induction of early apoptosis on the cell population.

Recovery of early apoptotic cells was further tested, for example, following 24 hours of storage at 2-8°C, for stability purposes, during which an average cell loss of 3-4.7% was measured, regardless of manufacturing conditions, with favorable results for cells that were both frozen and thawed with media containing anticoagulant (0.2±0.4% cell loss following 24 hours of FP storage), suggesting that addition of anticoagulant is critical during freezing and thawing, but once finally formulated, the early apoptotic cell population is stable. Extended time point studies showed this stability to at least 72 hours.

Apoptosis and viability, as well as cell composition of the FP product were not significantly affected by the addition of anticoagulant at the freezing and/or thawing stage. Values measured from a wide variety of characteristics were similar, indicating the ACDhep did not change the early apoptotic cell characteristics and the final product met the acceptance criteria of ≥40% apoptotic cells.

The assay used to test apoptotic cells potency was based on immature dendritic cells (iDCs), DCs that are characterized by functions such as phagocytosis, antigen presentation, and cytokine production.

The HLA-DR (MHC class II) membrane molecule and co-stimulatory molecule CD86 were selected as markers to detect the tolerogenic effects of antigen-presenting cells (APCs). Using flow cytometry, changes in expression of HLA-DR and CD86 on iDCs were measured following stimulation with LPS, as well as in the presence of the early apoptotic cell population manufactured with- or without anticoagulant and stimulated with LPS. Early apoptotic cell populations were offered to DCs in ascending ratios of 1:2, 1:4, and 1:8 iDCs : early apoptotic cell population. As presented in Table 6, it was shown that early apoptotic cell population enhanced the tolerogenic effect over stimulated DCs in a dose-dependent manner, with slightly better results for early apoptotic cell population manufactured with anticoagulant both at freezing and apoptosis induction.

Taken together, it was concluded that addition of anticoagulant to both freezing and apoptosis media is of high importance to increase cell recovery and avoid massive cell loss due to aggregates, and to avoid in many cases a second round of apheresis from a donor. It was shown that all cells met acceptance criteria for the validated FP, indicating that the addition of anticoagulant does not impair the FP.

### EXAMPLE 2: PREPARATION AND USE OF POOLED APOPTOTIC CELL

*Objective:* Produce an irradiated multiple donor single apoptotic cell infusion (a pooled mononuclear irradiated apoptotic cell preparation).

*Methods:* Apoptotic cells were prepared as per Example 1 above, except that in the current experiments, preparation was done simultaneously from multiple (4) donors. Following preparation from 4 donors, the cell preparations were combined at the last step (prior to irradiation), irradiated immediately after, and were ready for use. Irradiation was at 25 Gy.

### Results and Summary:

Analysis in a GvHD mouse model showed that the single infusion of multiple-donor irradiated apoptotic cells successfully and significantly improved life expectancy in a mouse model of GvHD. (Data not shown)

### REFERENCE EXAMPLE 3: Preparation of Apoptotic Cell Supernatant

*Objective:* To obtain a supernatant from early-apoptotic cells and monocytes/ macrophages/dendritic cells.

*Methods:* Preparation from apoptotic cells: CD14+ monocytes and other mononuclear enriched white blood cells were cultured and triggered to undergo apoptosis. The number of apoptotic cells was between 1 to 100 million cells per well in a 12-well plate. In some instances 8 million cells were cultured per well. After incubation for 24, 36, and 48 hours, the cells were centrifuge (290g, 4 degrees Celsius, 10 minutes). The supernatant was collected and frozen in aliquots at -80 degrees Celsius until use.

*Methods:* Preparation from monocytes/macrophages/dendritic cells and apoptotic cells: CD14+ monocytes were cultured with apoptotic cells as prepared above at a ratio of 1:16, for 24h or 36 h or 48 h. The number of monocytes was: 0.5 million cells per well in a 12-well plate and the number of apoptotic cells was: 8 million cells per well in a 12-well plate. After incubation for 24 hours the cells were centrifuge (290g, 4 degrees Celsius, 10 minutes). The supernatant was collected and frozen in aliquots at -80 degrees Celcius until use. Similar procedures could be performed at different ratios of monocytes:apoptotic cells and/or using other sources of cells derived from monocytes, such as different types of macrophages including M1/M2 and dendritic cells.

*Results:* The apoptotic supernatant prepared herein either from apoptotic mononuclear or from co-culturing monocytes/macrophages/dendritic cells with apoptotic mononuclear, was capable of down regulating pro-inflammatory cytokines under conditions of a cytokine storm (data not shown). These apoptotic supernatants, could in certain embodiments, be effectively used in methods disclosed herein for treating osteoarthritis.

### EXAMPLE 4: USE OF APOPTOIC CELLS TO TREAT OSTEOARTHRITIS

*Objective:* Treat osteoarthritis in a subject who was non-responsive to other therapies.

*Methods:* Early apoptotic cells prepared as described above in Example 1

Subject treated was a 70-year-old female with hypothyroidism. She presented with a 4-year inflammatory and erosive process of her right shoulder. In 2015, she experienced right upper extremity swelling and limited range-of-motion of the right shoulder, without known injury. Complete destruction of the humeral head on X-ray, significant inflammatory reaction on MRI, and significantly elevated erythrocyte sedimentation rate (ESR) and C-reactive protein (CRP) were seen. No serum autoantibodies or bacterial growth on needle aspiration of the joint were detected. ESR and CRP decreased with systemic steroids, without significant clinical improvement. After an uneventful total reverse shoulder arthroplasty, swelling, ESR and CRP elevations gradually subsided. Two years later the patient returned with shoulder swelling and significant CRP elevation. Repeated aspirations, debridements, and finally removal of the prosthesis, were performed, followed by long-term antibiotic treatment for the possibility of infection. Swelling and CRP levels were ameliorated only via continuous shoulder drain. During the last year she was hospitalized >9 months. Compassionate treatment using her own irradiated apoptotic cells was authorized by the Ethical Committee.

Irradiated, autologous early apoptotic cells were prepared as in Example 1 above (termed "Allocetra-OTS" in **Figure 2****).** The patient underwent leukapheresis to get the starting material and irradiated, early apoptotic cells were prepared from this starting material. The irradiated, early apoptotic cells (100 x 10⁶) were administrated by intra-joint injection in the right shoulder joint for 5 consecutive weeks. Specifically, each dose of cells administered contained 100 x 10⁶ ±20% irradiated, early apoptotic cells in 15 ml Ringer's Lactate Solution. Administration was by intra-joint infusion.

Measurement of cytokines and chemokines present in synovial flued was performed via a Luminex MAGPIX system (Luminex USA) using Milliplex software (Merck).

### Results:

After weekly intra-joint apoptotic cell infusions for 5 consecutive weeks, the patient improved dramatically, with significant reduction in shoulder swelling, redness, and tightness over 6 weeks. Fluid drainage from the shoulder declined from 150-250 ml/day to <60 ml/day. CRP declined from 7.34, pretreatment, to 0.49 (normal range ≤0.5). Synovial fluid samples were collected before treatment and weekly during/after treatment. Measurement of pro-inflammatory cytokines/chemokines related to monocyte, macrophage, dendritic cell, osteoclast, neutrophil, and T cell activation showed down regulation of IL-6, IL-8, IL-1β, IL-2, IL-15, IL-22, MIP-1β, IL-9 and TNFα, following 2 injections of irradiated early apoptotic cells **(****Figure 2). Figure 2** shows that most notably IL-22 (dysregulation of wound healing of synovial tissue), IL-8 (neutrophilic chemotactic factor), IL-6 (innate immunity), IL-9 (apoptosis prevention) and MIP-1-β (chronic inflammation) were downregulated.

Blood biochemistry and CBC were not significantly changed. An episode of drain-induced infection 3 weeks after the end of treatment was treated with antibiotics, debridement, and drain exchange. The drain was removed 3 months after treatment due to low fluid drainage and hospitalization was no longer required. At 6-month follow-up CRP remained low.

The effect observed in the first five weeks of treating the patient was maintained for nine months, where she remained at home and stable following the intervention. Surprisingly and unexpectedly, whereas Il-6 and perhaps IL-8 are cytokines that have been described in vanishing bone disease and could have been predicted to be elevated, this is the first description of high IL-22 which is related to synovial healing dysregulation. Neither its elevation nor the effect of treatment with early apoptotic cells were expected. The same can be said for IL-9 that can avoid apoptosis of inflammatory cells and therefore maintain chronic inflammation, whereas IL-9 down regulation may provoke activation induced cell death and terminate immune response. In addition, it was surprising that TNF was not elevated at all. In that regard anti-cytokine like anti-TNF and even anti IL-6 would not have been a good choices and only apoptotic cells had this global effect.

### Summary:

HLA-matched apoptotic cell infusion was previously reported to be safe and beneficial in prevention of graft-versus-host disease (Mevorach D, Zuckerman T, Reiner I, et al. (2014) "Single infusion of donor mononuclear early apoptotic cells as prophylaxis for graft-versus-host disease in myeloablative HLA-matched allogeneic bone marrow transplantation: a phase I/IIa clinical trial." Biol Blood Marrow Transplant., 20:58-65), but this is the first time autologous early apoptotic cells, specifically prepared as described herein, have been used as an intra-joint infusion.

The early apoptotic cell preparation prepared for treatment of this patient was autologous, yet one skilled in the art would appreciate that it would also have been possible to administer a non-autologous preparation of these early apoptotic cells to achieve the same results. Early apoptotic cells could have been obtained from either a third-party subject or could be a third-party pooled cell apoptotic preparation (allogeneic), followed by an irradiation step prior to administration and successfully treatments of osteoarthritis.

The mechanism of action of this treatment is suggested to be related to monocyte-, macrophage-, dendritic cell-, and osteoclast cell signaling (Trahtemberg U, Mevorach D. (2017) "Apoptotic cells induced signaling for immune homeostasis in macrophages and dendritic cells." Front Immunol., 8:1356).

Considering the conclusions of Grey et al., (ibid) that if the chosen route of administration does not deliver apoptotic cells to the spleen such that the apoptotic cells can interact with B cells, protection is unlikely to be elicited, it was highly unexpected that intra joint infusion to a non-autoimmune condition such as erosive osteoarthritis would be successful, as the apoptotic cells administered by intra joint infusion did not have any access to the spleen or to immune organ. Therefore, it was surprising and unexpected that the intra joint infusion terminated increased pro- and anti-inflammatory cytokine/chemokine release in the synovial fluid. Specially, the down regulation of high IL-22 which is related to synovial healing dysregulation and IL-9 that can avoid apoptosis of inflammatory cells and therefore maintain chronic inflammation whereas its down regulation may provoke termination of chronic inflammation.

As used herein, the singular form "a", "an" and "the" include plural references unless the context clearly dictates otherwise.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible sub ranges as well as individual numerical values within that range. For example, description of a range such as from 1 to 6 should be considered to have specifically disclosed sub ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between.

## Claims

1. A composition comprising a mononuclear-enriched early apoptotic cell population comprising more than 40% Annexin V and less than 15% PI positive cells, wherein said cells are irradiated following the induction of apoptosis, for use in a method of treating osteoarthritis in a human subject by administering the composition directly into a joint of said subject.

2. The composition for use according to claim 1, wherein treating osteoarthritis comprises pain reduction, reduction of inflammation, reduction of swelling, inhibition of progressive degeneration of articular cartilage, reduction of progressive degeneration of articular cartilage, improving a quality of life, or any combination thereof.

3. The composition for use according to any of claim 1 and claim 2, wherein said use increases movement in said joint, wherein increased movement comprises increased range of movement or increased movement with reduced pain, or a combination thereof.

4. The composition for use according to any of claims 1-3, wherein said joint comprises a synovial joint.

5. The composition for use according to any of claims 1-4, wherein said synovial joint comprises a knee joint, a hip joint, a shoulder joint, a joint between neck vertebrae, an elbow joint, an ankle joint, a wrist joint, a finger joint, a toe joint, or a thumb joint, a hand joint, a foot joint, or a combination thereof.

6. The composition for use according to any of claims 1-5, wherein said composition is formulated for direct administration into the joint as an infusion or injection.

7. The composition for use according to any of claims 1-6, wherein said early apoptotic cell population comprises a pooled population of early apoptotic cells.

8. The composition for use according to claim 7, wherein said pooled early apoptotic cell population comprises apoptotic cells prepared from a single donor or from multiple donor mononuclear cells.

9. The composition for use according to any of claims 1-8, wherein said administering comprises a single administration or multiple administrations of said early apoptotic cell population.

10. The composition for use according to claim 9, wherein said multiple administration comprises daily or weekly administrations.

11. The composition for use according to any of claims 1-10, wherein said composition is formulation to comprise a dose of between about 10 x 10⁶ ±20% to 1 x 10⁹ ±20% early apoptotic cells/kg subject.

## Patentansprüche

1. Eine Zusammensetzung enthaltend eine auf einkernige Zellen angereicherte Population früh-apoptotischer Zellen, mit mehr als 40% Annexin V-positiven und weniger als 15% Propidiumiodid-positiven Zellen, wobei die Zellen nach der Induktion der Apoptose bestrahlt werden, zur Verwendung in einem Verfahren zur Behandlung von Arthrose bei einem menschlichen Subjekt durch direkte Verabreichung der Zusammensetzung in ein Gelenk des Subjekts.

2. Eine Zusammensetzung zur Verwendung nach Anspruch 1, wobei die Behandlung der Arthrose eine Schmerzreduktion, Entzündungsreduktion, Schwellungsreduktion, Hemmung der fortschreitenden Degeneration des Gelenkknorpels, Reduzierung der fortschreitenden Degeneration des Gelenkknorpels, Verbesserung der Lebensqualität oder eine Kombination davon umfasst.

3. Eine Zusammensetzung zur Verwendung nach einem der Ansprüche 1 oder 2, wobei die Anwendung eine Erhöhung der Beweglichkeit des Gelenks bewirkt, wobei die erhöhte Beweglichkeit eine gesteigerte Bewegungsreichweite oder eine gesteigerte Bewegung bei reduzierten Schmerzen oder eine Kombination davon umfasst.

4. Eine Zusammensetzung zur Verwendung nach einem der Ansprüche 1 bis 3, wobei das Gelenk ein Synovialgelenk ist.

5. Eine Zusammensetzung zur Verwendung nach einem der Ansprüche 1 bis 4, wobei das Synovialgelenk ein Kniegelenk, Hüftgelenk, Schultergelenk, Gelenk zwischen Halswirbeln, Ellbogengelenk, Sprunggelenk, Handgelenk, Fingergelenk, Zehengelenk, Daumengelenk, Hand- oder Fußgelenk oder eine Kombination davon ist.

6. Eine Zusammensetzung zur Verwendung nach einem der Ansprüche 1 bis 5, wobei die Zusammensetzung zur direkten Verabreichung in das Gelenk als Infusion oder Injektion formuliert ist.

7. Eine Zusammensetzung zur Verwendung nach einem der Ansprüche 1 bis 6, wobei die Population früh-apoptotischer Zellen eine gepoolte Population von früh-apoptotischen Zellen umfasst.

8. Eine Zusammensetzung zur Verwendung nach Anspruch 7, wobei die gepoolte frühapoptotische Zellpopulation apoptotische Zellen umfasst, die von einem einzelnen Spender oder von mononukleären Zellen mehrerer Spender hergestellt wurden.

9. Eine Zusammensetzung zur Verwendung nach einem der Ansprüche 1 bis 8, wobei die Verabreichung eine einzelne oder multiple Verabreichung der früh-apoptotischen Zellpopulation umfasst.

10. Eine Zusammensetzung zur Verwendung nach Anspruch 9, wobei die multiple Verabreichung tägliche oder wöchentliche Verabreichungen umfasst.

11. Eine Zusammensetzung zur Verwendung nach einem der Ansprüche 1 bis 10, wobei die Zusammensetzung so formuliert ist, dass sie eine Dosierung von etwa 10 x 10⁶ +/- 20% bis 1 x 10⁹ +/- 20% früh-apoptotischer Zellen pro Kilogramm Körpergewicht des Subjekts umfasst.

## Revendications

1. Composition comprenant une population de cellules apoptotiques précoces enrichies en cellules mononucléaires comprenant plus de 40 % de cellules positives à l'annexine V et moins de 15 % de cellules positives au PI, dans laquelle lesdites cellules sont irradiées après l'induction de l'apoptose, pour utilisation dans un procédé de traitement de l'ostéoarthrite chez un sujet humain en administrant la composition directement dans une articulation dudit sujet.

2. Composition pour utilisation selon la revendication 1, dans laquelle le traitement de l'ostéoarthrite comprend la réduction de la douleur, la réduction de l'inflammation, la réduction du gonflement, l'inhibition de la dégénérescence progressive du cartilage articulaire, la réduction de la dégénérescence progressive du cartilage articulaire, l'amélioration de la qualité de vie, ou toute combinaison de celles-ci.

3. Composition pour utilisation selon l'une quelconque des revendications 1 et 2, dans laquelle ladite utilisation augmente le mouvement dans ladite articulation, dans laquelle l'augmentation du mouvement comprend l'augmentation de l'amplitude du mouvement ou l'augmentation du mouvement avec une réduction de la douleur, ou une combinaison de celles-ci.

4. Composition pour utilisation selon l'une quelconque des revendications 1 à 3, dans laquelle ladite articulation comprend une articulation synoviale.

5. Composition pour utilisation selon l'une quelconque des revendications 1 à 4, dans laquelle ladite articulation synoviale comprend une articulation du genou, une articulation de la hanche, une articulation de l'épaule, une articulation entre des vertèbres du cou, une articulation du coude, une articulation de la cheville, une articulation du poignet, une articulation du doigt, une articulation de l'orteil ou une articulation du pouce, une articulation de la main, une articulation du pied, ou une combinaison de celles-ci.

6. Composition pour utilisation selon l'une quelconque des revendications 1 à 5, dans laquelle ladite composition est formulée pour être administrée directement dans l'articulation sous la forme d'une perfusion ou injection.

7. Composition pour utilisation selon l'une quelconque des revendications 1 à 6, dans laquelle ladite population de cellules apoptotiques précoces comprend une population regroupée de cellules apoptotiques précoces.

8. Composition pour utilisation selon la revendication 7, dans laquelle ladite population de cellules apoptotiques précoces regroupées comprend des cellules apoptotiques préparées à partir d'un seul donneur ou de cellules mononucléaires de donneurs multiples.

9. Composition pour utilisation selon l'une quelconque des revendications 1 à 8, dans laquelle l'administration comprend une administration unique ou des administrations multiples de ladite population de cellules apoptotiques précoces.

10. Composition pour utilisation selon la revendication 9, dans laquelle ladite administration multiple comprend des administrations quotidiennes ou hebdomadaires.

11. Composition pour utilisation selon l'une quelconque des revendications 1 à 10, dans laquelle ladite composition est formulée pour comprendre une dose d'environ 10 x 10⁶ ±20 % à 1 x 10⁹ ±20 % de cellules apoptotiques précoces/kg de sujet.
